# EUROPEAN PATENT SPECIFICATION

(11) **EP 3 019 074 B1**
(45) Date of publication and mention of the grant of the patent: **29.08.2018**
(21) Application number: 14847625.2
(22) Date of filing: 30.09.2014
(51) Int. Cl.: A61B 5/00, A61K 31/192, A61P 25/28, A61K 31/22

(54) **DIAGNOSING, GRADING, MONITORING, AND TREATING HEPATIC ENCEPHALOPATHY**
DIAGNOSE, EINSTUFUNG, ÜBERWACHUNG UND BEHANDLUNG VON HEPATISCHER ENZEPHALOPATHIE
DIAGNOSTIC, CLASSEMENT, SURVEILLANCE ET TRAITEMENT D'ENCÉPHALOPATHIE HÉPATIQUE

(30) Priority: 30.09.2013 US 201361884922 P
(43) Date of publication of application: 18.05.2016
(73) Proprietor: Horizon Therapeutics, LLC, Lake Forest IL 60045 (US)
(72) Inventor: SCHARSCHMIDT, Bruce, San Francisco, California 94127 (US); MOKHTARANI, Masoud, Walnut Creek, California 94598 (US); JUREK, Marzena, Brisbane, California 94005 (US)
(74) Representative: Carpmaels & Ransford LLP
(86) International application number: PCT/US2014/058489
(87) International publication number: WO 2015/048818

(56) References cited:
- WO-A1-2006/056794
- US-A1- 2005 273 359
- US-A1- 2010 016 207
- US-B1- 8 404 215
- US-B2- 8 094 521
- MARWAN GHABRIL ET AL: "Glycerol Phenylbutyrate in Patients With Cirrhosis and Episodic Hepatic Encephalopathy: A Pilot Study of Safety and Effect on Venous Ammonia Concentration", CLINICAL PHARMACOLOGY IN DRUG DEVELOPMENT, vol. 2, no. 3, 16 March 2013 (2013-03-16), pages 278-284, XP055266459, ISSN: 2160-763X, DOI: 10.1002/cpdd.18
- ROCKEY DON C ET AL: "Randomized, controlled, double blind study of glycerol phenylbutyrate in patients with cirrhosis and episodic hepatic encephalopathy", HEPATOLOGY, vol. 56, no. Supplement 1, October 2012 (2012-10), page 248A, XP008179955, ISSN: 0270-9139, DOI: 10.1002/HEP.26040 [retrieved on 2012-10-01]
- KHUNGAR V ET AL: "Management of Overt Hepatic Encephalopathy", CLINICS IN LIVER DISEASE 2012 W.B. SAUNDERS USA, vol. 16, no. 1, February 2012 (2012-02), pages 73-89, XP008179943, ISSN: 1089-3261
- S. SUSHMA ET AL: "Sodium benzoate in the treatment of acute hepatic encephalopathy: A double-blind randomized trial", HEPATOLOGY, vol. 16, no. 1, 1 July 1992 (1992-07-01), pages 138-144, XP55266997, USA ISSN: 0270-9139, DOI: 10.1002/hep.1840160123
- J. S. BAJAJ ET AL: "Review article: the design of clinical trials in hepatic encephalopathy - an International Society for Hepatic Encephalopathy and Nitrogen Metabolism (ISHEN) consensus statement", ALIMENTARY PHARMACOLOGY & THERAPEUTICS., vol. 33, no. 7, 9 February 2011 (2011-02-09), pages 739-747, XP055266560, GB ISSN: 0269-2813, DOI: 10.1111/j.1365-2036.2011.04590.x
- MUNOZ, SJ.: 'Hepatic Encephalopathy''.' THE MEDICAL CLINICS OF NORTH AMERICA . vol. 92, 2008, pages 797 - 799, XP008116796
- CORDOBA, J.: 'New assessment of hepatic encephalopathy''.' JOURNAL OF HEPATOLOGY vol. 54, 2011, page 1030, 1032, 1038, XP028192163

## Description

### BACKGROUND

Hepatic encephalopathy (HE) refers to a spectrum of neurologic signs and symptoms believed to result from increased blood ammonia levels, which frequently occur in subjects with cirrhosis or certain other types of liver disease. Subjects with HE typically show altered mental status ranging from subtle changes to coma, features similar to those in subjects with urea cycle disorders (UCDs). The clinical manifestations of HE are not constant from day to day. Instead, the signs and symptoms of HE may periodically worsen, resulting in HE episodes (also known as HE events). HE episodes may occur in the setting of chronic liver disease (cirrhosis) due to all etiologies, as well as due to acute liver failure and spontaneous portosystemic shunts. Many if not most HE episodes occur when the patient is at home or in some setting other than the health care provider's office or clinical setting. Thus, it is important that the caregivers of patients with HE be familiar with the manifestations of HE episodes and be able to report them to the provider in an accurate and systematic manner. Maewan Ghabril et al (Clinical Pharmacology in Drug Development, vol. 2, no. 3, 16 March 2013, pages 278-284) disclose a clinical trial showing the beneficial effects of glycerol phenylbutyrate (GPB, HPN-100) in the treatment of patients with overt hepatic encephalopathy who experienced two or more documented episodes of West-Haven Grade ≥ 2 in the prior six months and at least 1 episode which occurred within three months of randomization.

### SUMMARY

Disclosed herein are novel grading systems for determining whether a subject is experiencing an overt HE episode and for grading an HE episode. Also disclosed herein are methods, instruments, and systems for treating an HE episode and monitoring HE episode treatment that utilize these grading systems. Also disclosed are methods of determining whether a subject is experiencing an overt HE episode by determining (a) whether the subject has been disoriented as to time, place, or person for at least one hour, (b) determining whether the subject has been lethargic for at least one hour and is exhibiting asterixis, and/or (c) determining that the subject is incapable of being assessed due to disorientation as to time, place, and person, somnolence, or coma, wherein the subject is classified as experiencing an overt HE episode if they meet any one of the criteria set forth in (a), (b), or (c). In a disclosure, if the subject meets any one of the criteria set forth in (a), (b), or (c), a therapeutic intervention as described herein may be administered to the subject. In a disclosure, if the therapeutic intervention is administered at a dosage sufficient to maintain the subject's fasting blood ammonia level at or below a specified threshold of 1.5 times the upper limit of normal. In a disclosure, the therapeutic intervention is a nitrogen scavenging drug. In a disclosure, the nitrogen scavenging drug is selected from the group consisting of a PAA prodrug and sodium benzoate. In a disclosure, the PAA prodrug is selected from the group consisting of glyceryl tri-[4-phenylbutyrate] (HPN-100), phenylbutyric acid (PBA), sodium PBA (NaPBA), and a combination of two or more of HPN-100, PBA, and NaPBA.

Disclosed herein are methods of determining whether a subject is experiencing a grade 2, 3, or 4 HE episode. In a disclosure, the subject is assessed for a grade 2 HE episode by (a) determining whether the subject has been disoriented as to time, place, or person for at least one hour and/or (b) determining whether the subject has been lethargic for at least one hour and is exhibiting asterixis, wherein the subject is classified as experiencing at least a grade 2 HE episode if they meet the criteria set forth in either (a) or (b). In a disclosure, the subject is assessed for a grade 3 HE episode by (c) determining whether the subject has been disoriented as to time, place, and person for at least one hour and/or (d) determining whether the subject has been somnolent for at least one hour, wherein the subject is classified as experiencing at least a grade 3 HE episode if they meet the criteria set forth in either (c) or (d). In a disclosure, the subject is assessed for a grade 4 HE episode by (e) determining whether the subject is comatose, wherein the subject is classified as experiencing a grade 4 HE episode if they meet the criteria set forth in (e). In a disclosure, if the subject meets any one of the criteria set forth in either (a) or (b), (c) or (d), or (e), then a therapeutic intervention as described herein may be administered to the subject. In a disclosure, the therapeutic intervention is administered at a dosage sufficient to maintain the subject's fasting blood ammonia level at or below a specified threshold of 1.5 times the upper limit of normal. In a disclosure, the therapeutic intervention is a nitrogen scavenging drug. The nitrogen scavenging drug is selected from the group consisting of a PAA prodrug and sodium benzoate. In a disclosure, the PAA prodrug is selected from the group consisting of glyceryl tri-[4-phenylbutyrate] (HPN-100), phenylbutyric acid (PBA), sodium PBA (NaPBA), and a combination of two or more of HPN-100, PBA, and NaPBA.

Disclosed herein are methods of treating an overt HE episode in a subject in need thereof by (a) determining whether the subject has been disoriented as to time, place, or person for at least one hour; (b) determining whether the subject has been lethargic for at least one hour and is exhibiting asterixis; and/or (c) determining that the subject is incapable of being assessed due to disorientation as to time, place, and person, somnolence, or coma, then administering a therapeutic intervention if the subject meets any one of the criteria set forth in (a), (b), or (c). In a disclosure, the therapeutic intervention is administered at a dosage sufficient to maintain the subject's fasting blood ammonia level at or below a specified threshold of 1.5 times the upper limit of normal. In a disclosure, the therapeutic intervention is a nitrogen scavenging drug. In a disclosure, the nitrogen scavenging drug is selected from the group consisting of a PAA prodrug and sodium benzoate. In a disclosure, the PAA prodrug is selected from the group consisting of glyceryl tri-[4-phenylbutyrate] (HPN-100), phenylbutyric acid (PBA), sodium PBA (NaPBA), and a combination of two or more of HPN-100, PBA, and NaPBA.

Disclosed herein are methods of treating an HE episode by (a) determining whether a subject is experiencing at least a grade 2 HE episode by (i) determining whether the subject has been disoriented as to time, place, or person for at least one hour and/or (ii) determining whether the subject has been lethargic for at least one hour and is exhibiting asterixis, wherein the subject is classified as experiencing at least a grade 2 HE episode if they meet the criteria set forth in either (i) or (ii); (b) determining whether a subject is experiencing at least a grade 3 HE episode by (iii) determining whether the subject has been disoriented as to time, place, and person for at least one hour and/or (iv) determining whether the subject has been somnolent for at least one hour, wherein the subject is classified as experiencing at least a grade 3 HE episode if they meet the criteria set forth in either (iii) or (iv); and (c) determining whether a subject is experiencing at least a grade 3 HE episode by (v) determining whether the subject is comatose, wherein the subject is classified as experiencing a grade 4 HE episode if they meet the criteria set forth in (v); then administering a therapeutic intervention if the subject is classified as experiencing a grade 2, 3, or 4 HE episode under steps (a), (b), or (c). In a disclosure, the therapeutic intervention is administered at a dosage sufficient to maintain the subject's fasting blood ammonia level at or below a specified threshold of 1.5 times the upper limit of normal. In a disclosure, the therapeutic intervention is a nitrogen scavenging drug. In a disclosure, the nitrogen scavenging drug is selected from the group consisting of a PAA prodrug and sodium benzoate. In a disclosure, the PAA prodrug is selected from the group consisting of glyceryl tri-[4-phenylbutyrate] (HPN-100), phenylbutyric acid (PBA), sodium PBA (NaPBA), and a combination of two or more of HPN-100, PBA, and NaPBA.

Disclosed herein are tools for screening a subject in need thereof for HE symptoms, the tools including (a) a first set of steps comprising one or more steps selected from (i) determining whether the subject has unusual difficulty speaking, (ii) determining whether the subject is exhibiting unusual behavior, and (iii) determining whether the subject is more forgetful or confused than usual, and (b) a second set of steps comprising one or more steps selected from (iv) determining whether the subject can stay awake when being spoken to, (v) determining whether the subject is disoriented as to person, (vi) determining whether the subject is disoriented as to place, and (vii) determining whether the subject is disoriented as to time. In a disclosure the second set of steps is performed only if one or more criteria from the first set of steps are met. In a disclosure, a user is instructed to contact a physician if one or more criteria from the second set of steps are met. In a disclosure, the user is a caregiver of the subject in need thereof. In a disclosure the steps of the tool are provided in a questionnaire format. In a disclosure the tool is provided in an electronic format with a branching logic algorithm. In a disclosure the tool is provided on a web-enabled device. In a disclosure, the first and, if necessary, second set of steps are performed on a daily basis to monitor the subject. In a disclosure daily reminders are electronically sent to the caregiver at the same time each day to remind the caregiver to use the tool. In a disclosure, the subject in need thereof is administered a therapeutic intervention if one or more criteria are met from the second set of steps. In a disclosure, the therapeutic intervention may be any therapeutic intervention as described herein. In a disclosure, the subject in need thereof is administered a therapeutic intervention according to a physician's recommendations. In a disclosure, if the subject was previously administered a dosage of lactulose, the dosage of lactulose is increased and the increased dosage of lactulose is administered to the subject. In a disclosure, the therapeutic intervention is administered at a dosage sufficient to maintain the subject's fasting blood ammonia level at or below a specified threshold of 1.5 times the upper limit of normal. In a disclosure the therapeutic intervention is a nitrogen scavenging drug. In a disclosure the nitrogen scavenging drug is selected from the group consisting of a PAA prodrug and sodium benzoate. In a disclosure the PAA prodrug is selected from the group consisting of glyceryl tri-[4-phenylbutyrate] (HPN-100), phenylbutyric acid (PBA), sodium PBA (NaPBA), and a combination of two or more of HPN-100, PBA, and NaPBA.

Provided herein in a disclosure are tools for screening a subject in need thereof for HE symptoms, the tools comprising one or more of the following steps (i) determining whether the subject can stay awake when being spoken to, (ii) determining whether the subject is disoriented as to person, (iii) determining whether the subject is disoriented as to place, and (iv) determining whether the subject is disoriented as to time. In a disclosure a user is instructed to contact a physician if one or more criteria in steps (i)-(iv) are met. In a disclosure, the user is a caregiver of the subject in need thereof. In a disclosure, steps (i)-(iv) are performed because the subject is exhibiting unusual mental or physical behavior, or a combination thereof. In a disclosure, the subject in need thereof is administered a therapeutic intervention if one or more criteria in steps (i)-(iv) are met. In a disclosure, the subject in need thereof is administered a therapeutic intervention according to the physician's recommendation if one or more criteria in steps (i)-(iv) are met. In a disclosure, the therapeutic intervention may be any therapeutic intervention as described herein. In a disclosure, if the subject was previously administered a dosage of lactulose, the dosage of lactulose is increased and the increased dosage of lactulose is administered to the subject. In a disclosure, the therapeutic intervention is administered at a dosage sufficient to maintain the subject's fasting blood ammonia level at or below a specified threshold of 1.5 times the upper limit of normal. In a disclosure, the therapeutic intervention is a nitrogen scavenging drug. In a disclosure, the nitrogen scavenging drug is selected from the group consisting of a PAA prodrug and sodium benzoate. In a disclosure, the PAA prodrug is selected from the group consisting of glyceryl tri-[4-phenylbutyrate] (HPN-100), phenylbutyric acid (PBA), sodium PBA (NaPBA), and a combination of two or more of HPN-100, PBA, and NaPBA. In a disclosure, the steps of the tool are provided in a questionnaire format. In certain embodiments, the tool is provided in an electronic format with a branching logic algorithm. In a disclosure, the tool is provided on a web-enabled device.

Provided herein in a disclosure are methods of treating HE symptoms in a subject in need thereof, the methods comprising (a) a first set of steps comprising one or more steps selected from (i) determining whether the subject has unusual difficulty speaking, (ii) determining whether the subject is exhibiting unusual behavior, and (iii) determining whether the subject is more forgetful or confused than usual; and (b) a second set of steps comprising one or more steps selected from (iv) determining whether the subject can stay awake when being spoken to, (v) determining whether the subject is disoriented as to person, (vi) determining whether the subject is disoriented as to place, and (vii) determining whether the subject is disoriented as to time. In a disclosure, the second set of steps are performed only if one or more criteria from the first set of steps are met. In a disclosure, a physician is contacted if one or more criteria are met from the second set of steps. In a disclosure, a caregiver of the subject in need thereof performs the first and, if necessary, second set of steps. In a disclosure, the steps of the method are provided to the caregiver using the screening tool as provided herein. In a disclosure, the steps are provided in a questionnaire format. In a disclosure, the steps are provided in an electronic format with a branching logic algorithm. In a disclosure, the steps are provided on a web-enabled device. In a disclosure, the first and, if necessary, second set of steps are performed on a daily basis to monitor the subject. In a disclosure, daily reminders are electronically sent to the caregiver at the same time each day to remind the caregiver to use the tool. In a disclosure, the methods include a step of administering a therapeutic intervention to the subject in need thereof if one or more criteria from the second set of steps are met. In a disclosure, the subject in need thereof is administered a therapeutic intervention according to the physician's recommendation if one or more criteria are met from the second set of steps. In a disclosure, the therapeutic intervention may be any therapeutic intervention as described herein. In a disclosure, if the subject was previously administered a dosage of lactulose, the dosage of lactulose is increased and the increased dosage of lactulose is administered to the subject. In a disclosure, the therapeutic intervention is administered at a dosage sufficient to maintain the subject's fasting blood ammonia level at or below a specified threshold of 1.5 times the upper limit of normal. In a disclosure, the therapeutic intervention is a nitrogen scavenging drug. In certain embodiments, the nitrogen scavenging drug is selected from the group consisting of a PAA prodrug and sodium benzoate. In a disclosure, the PAA prodrug is selected from the group consisting of glyceryl tri-[4-phenylbutyrate] (HPN-100), phenylbutyric acid (PBA), sodium PBA (NaPBA), and a combination of two or more of HPN-100, PBA, and NaPBA.

Provided herein in a discloosure are methods of treating HE symptoms in a subject in need thereof, the methods comprising one or more of the following steps (i) determining whether the subject can stay awake when being spoken to, (ii) determining whether the subject is disoriented as to person, (iii) determining whether the subject is disoriented as to place, and (iv) determining whether the subject is disoriented as to time. In certain embodiments, a physician is contacted if one or more of the criteria in steps (i)-(iv) are met. In certain embodiments, a caregiver of the subject in need thereof performs the one or more of steps (i)-(iv). In a disclosure, the one or more of steps (i)-(iv) are performed because the subject is exhibiting unusual mental or physical behavior, or a combination thereof. In a disclosure, the steps of the method are provided to the caregiver using the screening tool as provided herein. In a disclosure, the steps are provided in a questionnaire format. In a disclosure, the steps are provided in an electronic format with a branching logic algorithm. In a disclosure, the steps are provided on a web-enabled device. In a disclosure, the methods further comprise administering a therapeutic intervention to the subject in need thereof if one or more criteria in steps (i)-(iv) are met. In a disclosure, the methods further comprise administering a therapeutic intervention according to the physician's recommendation to the subject in need thereof if one or more criteria in steps (i)-(iv) are met. In a disclosure, if the subject was previously administered a dosage of lactulose, the dosage of lactulose is increased and the increased dosage of lactulose is administered to the subject. In a disclosure, the therapeutic intervention is administered at a dosage sufficient to maintain the subject's fasting blood ammonia level at or below a specified threshold of 1.5 times the upper limit of normal. In a disclosure, the therapeutic intervention is a nitrogen scavenging drug. In a disclosure, the nitrogen scavenging drug is selected from the group consisting of a PAA prodrug and sodium benzoate. a disclosure, the PAA prodrug is selected from the group consisting of glyceryl tri-[4-phenylbutyrate] (HPN-100), phenylbutyric acid (PBA), sodium PBA (NaPBA), and a combination of two or more of HPN-100, PBA, and NaPBA.

Also provided herein in disclosure are kits comprising the screening tools as described herein to assist a caregiver with monitoring, documenting, and/or treating HE symptoms in a subject in need thereof.

### DETAILED DESCRIPTION

According to present invention, glyceryl tri-[4-phenylbutyrate] (HPN-100) is provided for use in a method of treating an overt hepatic encephalopathy (HE) episode in an adult or pediatric human subject suspected to be currently experiencing an HE episode, wherein the glyceryl tri-[4-phenylbutyrate] (HPN-100) is administered to the subject if the subject is classified as experiencing an overt HE episode; wherein the subject is classified as experiencing an overt HE episode if the subject meets only one of the criteria set forth in (a), (b), or (c):
(a) determining whether the subject has been disoriented as to time, place, or person for at least one hour;
(b) determining whether the subject has been lethargic for at least one hour and is exhibiting asterixis; or
(c) determining that the subject is incapable of being assessed due to disorientation as to time, place, and person, somnolence, or coma,
wherein the glyceryl tri-[4-phenylbutyrate] (HPN-100) is administered at a dosage sufficient to maintain the subject's fasting blood ammonia level at or below a specified threshold of 1.5 times the upper limit of normal.

In a certain embodiment, the upper limit of normal for blood ammonia is 35 µmmol/L.

The following description of the invention is intended to illustrate various embodiments of the invention. The invention is defined by the claims. Any subject-matters falling outside the scope of the claims is provided for information only.

HE episodes can be precipitated (i.e., arising in response to a known trigger), spontaneous (i.e., arising without an identifiable trigger), and/or recurrent (occurring multiple times within a single year). HE episodes are routinely divided into grades or classes based on an assessment of a subject's physical and mental state. Grade 1 HE (sometimes also referred to as covert HE) is generally mild and undetectable by routine clinical examination. Grade 2 to 4 HE, also known as overt HE episodes, are more severe and typically require therapeutic intervention. The level of therapeutic intervention may vary depending on episode severity and other factors. Prompt identification and classification of overt HE episodes is critical for initiation of appropriate therapy to prevent advancement to more severe grades.

The systems used to grade HE episodes have evolved over several decades. The two major diagnostic components of HE episode grading generally are altered mental status and generalized motor disturbance, both of which can be highly subjective. The way in which these two components are evaluated differs between systems, and the general trend in grading systems in recent years has been towards more operationally focused criteria in order to minimize subjectivity and inter-observer variability.

The original HE grading system was developed in the 1950s (Parsons-Smith 1957). In this system, a subject was classified as having a grade 1 HE episode if they exhibited a trivial lack of awareness, euphoria or apathy in the absence of unequivocal neurological abnormalities, and impaired performance of simple neuropsychiatric tests, including the construction of a 5-pointed star and the ability to add or subtract serial 7's. A subject was classified as having a grade 2 HE episode if they exhibited obvious personality changes with definite neurologic abnormality (with a flapping tremor being the most characteristic), but with a gross facade of personality preserved. A subject was classified as having a grade 3 HE episode if they exhibited advanced confusion and disorientation, and a grade 4 HE episode if they were stuporose but responding to stimuli.

The next grading system to be developed was the original West Haven Scale, also referred to as the Conn Score Scale (Conn 1977). In this system, a subject is classified as having a grade 1 HE episode if they exhibit a trivial lack of awareness, euphoria or anxiety, shortened attention span, and impaired performance of addition or subtraction. A subject is classified as having a grade 2 HE episode if they exhibit lethargy, disorientation with regard to time, obvious personality change, and inappropriate behavior. A subject is classified as having a grade 3 HE episode if they exhibit somnolence to semi-stupor, are responsive to stimuli, are confused, exhibit gross disorientation, and exhibit bizarre behavior. A subject is classified as having a grade 4 HE episode if they are comatose.

In the Practice Guidelines system (Blei 2001), a subject is classified as having a grade 1 HE episode if they exhibit a trivial lack of awareness, shortened attention span, impaired addition or subtraction, hypersomnia, insomnia or inversion of sleep pattern, euphoria or depression, and asterixis. A subject is classified as having a grade 2 episode if they exhibit lethargy or apathy, disorientation, inappropriate behavior, slurred speech, and obvious asterixis. A subject is classified as having a grade 3 HE episode if they exhibit gross disorientation, bizarre behavior, and semistupor to stupor, with asterixis generally absent. A subject is classified as having a grade 4 HE episode if they are comatose.

The 1998 Congress of Gastroenterology system (Ferenci 2002) represented the first consensus panel to standardize HE grading terminology. In this system, a subject is classified as having a grade 1 HE episode if they exhibit a trivial lack of awareness, euphoria or anxiety, shortened attention span, and impaired performance of addition. A subject is classified as having a grade 2 HE episode if they exhibit lethargy or apathy, minimal disorientation of time and place, subtle personality change, inappropriate behavior, and impaired performance of subtraction. A subject is classified as having a grade 3 HE episode if they exhibited somnolence to semi-stupor but with responsiveness to verbal stimuli, confusion, and gross disorientation. A subject is classified as having a grade 4 HE episode if they are comatose (i.e., unresponsive to verbal or noxious stimuli).

In the Operative Definitions of West Haven system (Amodio 2004), a subject is classified as having a grade 1 HE episode if they are oriented as to time and place but 1) have 5 years or more of education and are unable to complete the trail-making test TMT-A in 120 seconds or 2) are unable to name more than eight animals in 120 seconds. A subject is classified as having a grade 2 HE episode if they are oriented as to place but disoriented as to time. Disorientation as to time under this system means that the subject gets three or more items wrong out of the day of the week, day of the month, month, or year. A subject is classified as having a grade 3 HE episode if they are disoriented as to time, exhibit a reduced Glasgow Coma Scale (GCS) score of 8 to 14, and are disoriented as to place. Disorientation as to place under this system means that the subject gets two or more items wrong out of their current state/country, region/county, city, place, or floor/ward. A subject is classified as having a grade 4 HE episode if they are unresponsive to pain stimuli (i.e., Glasgow Score under 8).

In the Hepatic Encephalopathy Scoring Algorithm (HESA) system (Hassanein 2007; Hassanein 2008; Hassanein 2010), a subject is classified as having a grade 1 HE episode if they exhibit sleep disturbances and tremor. A subject is classified as having a grade 2 HE episode if they exhibit lethargy, disorientation as to time, slurred speech, hyperactive reflexes, and inappropriate behavior. A subject is classified as having a grade 3 HE episode if they exhibit somnolence, confusion, disorientation as to place, bizarre behavior, and clonus/rigidity. A subject is classified as having a grade 4 HE episode if they exhibit no eye opening, no verbal response, and no reaction to simple commands.

In the International Society for Hepatic Encephalopathy and Nitrogen Metabolism (ISHEN) Consensus Statement system (Bajaj 2011), a subject is classified as having a grade 1 HE episode if they did not exhibit a change in mental state but did exhibit neuropsychometric/neuropsychologic abnormalities in the absence of disorientation and asterixis. A subject is classified as having an overt HE episode (i.e., grade 2 or higher) if they exhibit a change in mental state. A subject is classified as having a grade 2 HE episode if they exhibit a change in mental state and disorientation as to time. A subject is classified as having a grade 4 HE episode if they are comatose.

Additional grading systems include the Clinical Hepatic Encephalopathy Staging Scale (CHESS) (Ortiz 2007), which focuses exclusively on a subject's orientation, alertness, and ability to talk and respond to commands, and the Modified Orientation Log (MO-Log) (Salam 2012), which is adapted from a validated brain injury measure.

In 2014, joint practice guidelines using a data-supported approach were issued by the American Association for the Study of Liver Diseases and the European Association for the Study of the Liver pertaining to HE (Vilstrup 2014). These joint guidelines built on prior work (e.g. Ferenci 2002, Bajaj 2011) in that HE was again divided into covert (minimal and grade 1) and overt (grades 2-4) categories and causally classified as A (acute liver failure), B (portosystemic shunting or bypass) or C (cirrhosis). These guidelines also recognized the importance of Caregiver counseling, although no specific guidelines were provided.

As disclosed herein, an improved grading system has been developed for assessing whether a subject is experiencing or has recently experienced an overt (i.e., grade 2 or higher) HE episode. In this grading system, a subject is classified as currently or recently experiencing an overt HE episode if they (1) are disoriented as to time, place, or person; (2) are lethargic and have asterixis as evidenced by the occurrence of at least three flaps in a 30 second period; or (3) cannot be assessed due to disorientation as to time, place, and person, somnolence, or coma. A subject need only meet one of these three standards to be classified as currently or recently experiencing an overt HE episode. However, in certain embodiments a subject may meet more than one standard. For each criteria other than asterixis (i.e., disorientation, lethargy, somnolence, coma), the criteria must have been in place for at least one hour for the standard to be met.

In addition to determining whether a subject is currently experiencing or has recently experienced an overt HE episode, the grading system disclosed herein can be used to classify an HE episode as grade 2, 3, or 4. An HE episode is classified as grade 2 if the subject (1) exhibits disorientation as to time, place, or person or (2) exhibits asterixis and lethargy. An HE episode is classified as grade 3 if the subject (1) exhibits disorientation as to time, place, and person or (2) is somnolent. An HE episode is classified as grade 4 if the subject is comatose. For each criteria other than asterixis (i.e., disorientation, lethargy, somnolence, coma), the criteria must have been in place for at least one hour for the standard to be met. In certain embodiments, classification of an HE episode using the grading system provided herein is used to determine whether to initiate treatment and/or what type of treatment to administer.

A "subject" or "subject in need thereof' as used herein includes any adult or pediatric human suspected to be currently experiencing an HE episode, known to have or suspected to have experienced one or more HE episodes in the past, or deemed at risk of experiencing an HE episode in the future. In certain embodiments, a subject may be currently diagnosed with, previously been diagnosed with, suspected of having, or deemed at risk of developing impaired liver function due to one or more conditions, including for example cirrhosis. In certain embodiments, a subject may exhibit or may have previously exhibited elevated blood levels of one or more toxic substances, including for example ammonia. In certain embodiments, a subject may have a documented history of cirrhosis and HE, meaning that the subject has been diagnosed with cirrhosis and HE on at least one occasion based on observation and assessment by a medical professional or on caregiver input and/or review of medical records.

"Treating" or "treatment" as used herein with regard to an HE episode or HE symptoms may refer to terminating an HE episode, shortening the duration of an HE episode, lowering the grade of an HE episode (e.g., lowering a grade 4 HE episode to a grade 2 or 3 HE episode), reducing, eliminating, or shortening the duration of one or more symptoms associates with an HE episode, and/or reducing the likelihood of recurrence of an HE episode. In certain embodiments, treatment may result in a decrease in one or more toxic substances in the blood, including for example ammonia.

A "flap" as used herein refers to an inability to maintain a postural tone as evidenced by jerking movements of a subject's outstretched hands when bent upwards at the wrist. Flaps are sometimes alternatively referred to in the art as flapping tremors or liver flaps.

"Recently experiencing" as used herein with regard to an HE episode means that the subject is not currently experiencing an HE episode but has experienced at least one HE episode in the near past. For example, the subject may have experienced an HE episode within the past 6 hours, 12 hours, 1 day, 2 days, 3 days, 4 days, 5 days, 6 days, 1 week, 2 weeks, or 1 month. Alternatively, the subject may have experienced an HE episode in the time period since their last examination by a medical professional.

Methods of Grading and Treating HE. The grading system disclosed herein differs in several ways from previously developed grading systems.

First, the presently disclosed grading system requires the presence of only one (disorientation) or two (lethargy and asterixis) criteria to classify a subject capable of assessment as currently or recently experiencing an overt (i.e., grade 2 or higher) HE episode. Previously developed systems did not consider disorientation alone or the combination of lethargy and asterixis sufficient to classify a subject as having a grade 2 HE episode. This is advantageous because it requires clinicians to assess fewer criteria, thereby reducing variability and subjectivity. Previously developed grading systems that incorporate both lethargy and disorientation as criteria for a grade 2 HE episode require both of these criteria to be present. Similarly, previously developed grading systems that incorporate both asterixis and disorientation as a criteria for a grade 2 HE episode require both of these criteria to be present.

Second, the presently disclosed grading system requires all clinical criteria other than asterixis to persist for at least one hour. This further reduces subjectivity by providing a specific timeframe over which the criteria must be present.

Third, the presently disclosed grading system makes disorientation as to time, place, or person sufficient to classify a subject as currently or recently experiencing an overt HE episode. Previously disclosed grading systems either do not provide for this flexibility or are vague as to what type of disorientation is sufficient for an over HE episode.

Fourth, the presently disclosed grading system includes a specific requirement for assessment of asterixis, specifically the occurrence of at least three flaps in a 30 second period. This is a more precise requirement for asterixis than that provided in previously developed grading systems.

Fifth, the presently disclosed grading system provides precise operational definitions for the various criteria. This represents an improvement over previously disclosed systems, which frequently include ambiguous terminology.

Overall, the presently disclosed grading system provides a simpler and more objective system for identifying and grading HE episodes. The reduction in variability and subjectivity associated with previous systems translates to more rapid and precise treatment.

In certain embodiments of the grading system and related methods provided herein, whether a subject is disoriented as to time is determined by ascertaining whether the subject knows the current month, year, day of the week, and/or date of the month. In certain of these embodiments, a subject is classified as disoriented as to time if they either (1) do not know the year or (2) do not know two or more of the other items (i.e., month, day of week, date of month). In these embodiments, the presently disclosed grading system further differs from previously disclosed systems in that it requires only one or two criteria for assessment of disorientation as to time (either disorientation as to year or disorientation as to two or more of month, day of week, date of month). In certain embodiments, whether a subject knows the month, year, day, and/or date is determined by a medical professional (e.g., a medical doctor) in a clinical or non-clinical setting, and in certain of these embodiments the determination is made based on direct questioning of the subject. In other embodiments, the determination may be made based on caregiver input and/or review of medical records.

In certain embodiments of the grading system and related methods provided herein, whether a subject is disoriented as to place is determined by ascertaining whether the subject knows their current country, province/state, city/town, and/or type of place (e.g., hospital, house). In certain of these embodiments, a subject is classified as disoriented as to place if they do not know one or more of these items (i.e., country, province/state, city/town, place). In certain embodiments, whether a subject knows the country, province/state, city/town, place is determined by a medical professional in a clinical or non-clinical setting, and in certain of these embodiments the determination is made based on direct questioning of the subject. In other embodiments, the determination may be made based on caregiver input and/or review of medical records.

In certain embodiments of the grading system and related methods provided herein, whether a subject is disoriented as to person is determined by ascertaining whether the subject remembers and can state their own name and/or can recognize and name one or more family members or caregivers who are present. In certain of these embodiments, a subject is classified as disoriented as to person if they fail to meet one or both of these criteria. In certain embodiments, whether a subject remembers and can state their own name or recognize and name one or more family members or caregivers is determined by a medical professional in a clinical or non-clinical setting, and in certain of these embodiments the determination is made based on direct questioning of the subject. In other embodiments, the determination may be made based on caregiver input and/or review of medical records.

In the grading system and related methods provided herein, a subject is classified as currently or recently experiencing asterixis if they exhibit at least three flaps in a 30 second period. This determination can be made by a medical professional in a clinical or non-clinical setting or by a review of medical records. Similarly, the determination of whether a subject is comatose can be made by a medical professional in a clinical or non-clinical setting.

In certain embodiments of the grading system and related methods provided herein, whether a subject is lethargic is determined by ascertaining whether the subject is inattentive and sleepy but awakens when spoken to. If so, the subject is classified as lethargic. In certain embodiments, whether a subject is lethargic is determined by a medical professional in a clinical or non-clinical setting, and in certain of these embodiments the determination is made based on direct observation and/or questioning of the subject. In other embodiments, the determination may be made based on caregiver input and/or review of medical records.

In certain embodiments, whether a subject is somnolent is determined by ascertaining whether the subject is asleep but temporarily arousable in response to verbal or physical stimulation. In certain embodiments, whether a subject is somnolent is determined by a medical professional in a clinical or non-clinical setting, and in certain of these embodiments the determination is made based on direct observation and/or questioning of the subject. In other embodiments, the determination may be made based on review of medical records.

As noted above, all criteria of the current grading system that are specific for a certain grade (i.e., disorientation, lethargy, somnolence, coma) must be present for at least one hour for a subject to be classified as positive for that criteria. In certain embodiments, this determination is made based on two separate evaluations performed by the same medical professional or by two different medical professionals one or more hours apart. In other embodiments, the start of the occurrence of the criteria may be determined based on caregiver input and/or medical records, with the medical professional making the determination that the criteria is still present one or more hours later. In other embodiments, the entire determination may be based on caregiver input and/or medical records. For example, in certain embodiments a caregiver may attest that lethargy has been present in the subject for one hour or more. In certain embodiments, a separate assessment may nonetheless be carried out by a medical professional in a clinical or non-clinical setting. In other embodiments, caregiver input and/or medical records may be sufficient to establish the existence of one or more criteria.

Provided herein in certain embodiments are methods of determining whether a subject is experiencing or has recently experienced an overt HE episode using the grading system disclosed herein. Also provided herein are systems and instruments for applying this grading system to make such a determination, as well as methods of treating overt HE episodes that incorporate this grading system.

Provided herein in certain embodiments are methods of determining whether a subject is experiencing or has recently experienced a grade 2, 3, or 4 HE episode using the grading system disclosed herein. Also provided herein are systems and instruments for applying this grading system to make such a determination, as well as methods of treating grade 2, 3, or 4 HE episodes that incorporate this grading system.

Provided herein in certain embodiments are methods of classifying an HE episode in a subject as grade 2, 3, or 4 using the grading system disclosed herein. Also provided herein are systems and instruments for applying this grading system to classify an HE episode, as well as methods of treating HE episodes that incorporate this classification.

Provided herein in certain embodiments are methods for determining whether a subject is experiencing or has recently experienced an overt HE episode. In certain embodiments, these methods comprise (a) determining whether the subject has been disoriented as to time, place, or person for at least one hour, (b) determining whether the subject has been lethargic for at least one hour and exhibits asterixis, and/or (c) determining that the subject is incapable of being assessed due to disorientation as to time, place, and person, somnolence, or coma. In these embodiments, a subject is classified as currently or recently experiencing an overt HE episode if they meet the criteria set forth in (a), (b), or (c).

Provided herein in certain embodiments are methods for determining whether a subject is experiencing or has recently experienced at least a grade 2 HE episode. In certain embodiments, these methods comprise (a) determining whether the subject has been disoriented as to time, place, or person for at least one hour and/or (b) determining whether the subject has been lethargic for at least one hour and exhibits asterixis. In these embodiments, a subject is classified as currently or recently experiencing at least a grade 2 HE episode if they meet the criteria set forth in (a) or (b).

Provided herein in certain embodiments are methods for determining whether a subject is experiencing or has recently experienced at least a grade 3 HE episode. In certain embodiments, these methods comprise (a) determining whether the subject has been disoriented as to time, place, and person for at least one hour and/or (b) determining whether the subject has been somnolent for at least one hour, wherein the subject is classified as currently or recently experiencing at least a grade 3 HE episode if they meet the criteria set forth in either (a) or (b).

Provided herein in certain embodiments are methods for determining whether a subject is experiencing or has recently experienced a grade 4 HE episode. In certain embodiments, these methods comprise determining whether the subject is comatose, wherein the subject is classified as currently or recently experiencing a grade 4 HE episode if they meet this criteria.

Provided herein in certain embodiments are methods for classifying or grading an HE episode in a subject as grade 2, 3, or 4 using the criteria disclosed above for determining whether a subject is experiencing a grade 2, 3, or 4 HE episode. In certain embodiments, these methods comprise (a) determining whether the subject is experiencing at least a grade 2 HE episode; (b) if (a) is met, determining whether the subject is experiencing at least a grade 3 HE episode; and (c) if (b) is met, determining whether the subject is experiencing a grade 4 HE episode. In certain embodiments, each of these determinations is made in order. For example, before ascertaining whether the subject is experiencing a grade 3 or 4 episode, a subject is first evaluated for a grade 2 HE episode. In other embodiments, one or more of these steps may be skipped. For example, in certain embodiments, criteria for a grade 4 episode may be evaluated first. If the subject is classified as experiencing a grade 4 HE episode, evaluation of the criteria for grades 2 and 3 may be skipped. In still other embodiments, the order of the steps is reversed. In these embodiments, the subject is first evaluated for a grade 4 HE episode. If the criteria for a grade 4 HE episode are not met, the subject is evaluated for a grade 3 HE episode. If those criteria are not met, the subject is evaluated for a grade 2 HE episode.

In certain embodiments, the methods provided herein for classifying or grading an HE episode in a subject comprise (a) determining whether the subject is experiencing or has recently experienced at least a grade 2 HE episode by (i) determining whether the subject has been disoriented as to time, place, or person for at least one hour and/or (ii) determining whether the subject has been lethargic for at least one hour and exhibits asterixis, wherein the subject is classified as experiencing at least a grade 2 HE episode if they meet the criteria set forth in either (i) or (ii). In certain embodiments, the methods comprise (b) determining whether the subject is experiencing or has recently experienced at least a grade 3 HE episode by (iii) determining whether the subject has been disoriented as to time, place, and person for at least one hour and/or (iv) determining whether the subject has been somnolent for at least one hour, wherein the subject is classified as experiencing at least a grade 3 HE episode if they meet the criteria set forth in either (iii) or (iv). In certain embodiments, the methods comprise (c) determining whether the subject is experiencing or has recently experienced a grade 4 HE episode by (v) determining whether the subject is comatose, wherein the subject is classified as experiencing a grade 4 HE episode if they meet the criteria in (v).

In certain embodiments, the methods provided herein for identifying an overt HE episode or classifying an HE episode as grade 2, 3, or 4 can be deployed in a clinical trial setting. For example, the methods provided herein may be used to assess the efficacy of an experimental therapeutic intervention or to select subjects for inclusion in or removal from a clinical trial.

In certain embodiments, the methods provided herein for treating an HE episode comprise determining whether a subject is experiencing or has recently experienced an overt HE episode using the grading system provided herein, then administering one or more therapeutic interventions if the subject is classified as currently or recently experiencing an overt HE episode. Similarly, in certain embodiments the methods provided herein for treating an HE episode comprise determining whether a subject is experiencing or has recently experienced a grade 2, 3, or 4 HE episode using the grading system provided herein, then administering one or more therapeutic interventions if the subject is classified as experiencing a grade 2, 3, or 4 HE episode.

In certain embodiments, the therapeutic intervention may be a nitrogen scavenging or ammonia binding agent such as a phenylacetate (PAA) prodrug (e.g., PBA, sodium PBA (Buphenyl®), glycerol phenylbutyrate (glyceryl tri-[4-phenylbutyrate], HPN-100, Ravicti®)) or sodium benzoate, L-omithine, L-aspartate (LOLA), a minimally absorbable or non-absorbable antibiotic (e.g., Rifaximin (Xifaxan®) or a polymorph thereof, neomycin), dietary protein and/or vitamin supplementation, a minimally absorbable or non-absorbable disaccharide (e.g., lactulose, lactitol), probiotics, flumazenil acarbose, an antimicrobial agent, hypertonic saline, mannitol, dexamethasone, thiopentone, indomethacin, an antiepileptic drug, N-acetylcysteine (NAC), or any combination thereof. In other embodiments, the therapeutic intervention may be a non-drug intervention such as intubation and mechanical ventilation, sedation, enema, liver transplant, or a liver assist device.

In certain embodiments of the methods provided herein, the therapeutic intervention may differ depending on the grade assigned to the HE episode. For example, a subject identified as currently or recently experiencing a grade 4 HE episode may receive a more aggressive therapeutic intervention than a subject identified as experiencing a grade 2 or 3 HE episode. In certain embodiments, the therapeutic intervention may be selected based on its ability to target specific symptoms associated with a particular HE episode grade. In those embodiments where the therapeutic intervention is a drug, identification of a higher grade HE episode may result in administration of the same drug at a higher dosage or for a longer duration than would be administered for a lower grade HE episode, or it may result in administration of a more powerful drug or a combination of drugs. In those embodiments where the therapeutic intervention is not a drug, identification of a higher grade HE episode may result in the use of more aggressive therapeutic intervention options such as intubation for airway protection with or without mechanical ventilation. In certain embodiments, a subject may have previously been administered the same drug or a different drug for treatment of HE. A drug for use in treating an HE episode may be administered on a one-time basis or for a specific duration. Alternatively, the drug may be administered indefinitely or until a specific therapeutic benchmark is achieved, for example a cessation of HE episode, a decrease in likelihood of experiencing an HE episode, or a decrease in one or more symptoms associated with HE.

In certain embodiments of the treatment methods disclosed herein, the subject has previously received or is currently receiving one or more therapeutic interventions for HE. For example, a subject may have previously been administered or may currently be receiving one or more nitrogen scavenging drugs. In other embodiments, the subject has not received any therapeutic intervention for HE prior to the initial application of the grading system. In certain embodiments, the subject has been diagnosed with HE or with one or more HE episodes previously. In other embodiments, the subject has not been previously diagnosed with HE or an HE episode. In certain of these embodiments, the subject may be suspected of having experienced an HE episode previously, but without an official diagnosis.

In certain embodiments, the methods provided herein for determining whether a subject is experiencing or has recently experienced an HE episode and for grading HE episodes can be used to determine whether a subject is improving or worsening or to monitor and/or modify treatment. In certain of these embodiments, a subject that has previously been identified as having an HE episode is re-assessed using the grading system provided herein. If the subject is found to no longer be experiencing an overt HE episode or to be experiencing a lower grade HE episode than previously assessed, the subject is classified as improving. In those embodiments where the subject has received one or more therapeutic interventions in the interim between grading assessments, the therapeutic interventions may be classified as effective, and the subject may continue receiving the same dosage/type of therapeutic intervention. In certain embodiments, the subject may even begin receiving a decreased dosage. Alternatively, if the subject is found to still be experiencing an overt HE episode or to be experiencing the same or a higher grade HE episode than previously assessed, the subject is classified as not improving or worsening. In those embodiments where the subject has received one or more therapeutic interventions in the interim between grading assessments, the therapeutic interventions may be classified as ineffective, and the subject may be switched to higher dosages or more frequent administration of the same therapeutic interventions or to a more aggressive therapeutic intervention.

In certain embodiments, the therapeutic intervention may be administered at a dosage sufficient to maintain a subject's fasting blood ammonia level at or below a specified threshold level of 1.5 times the upper limit of normal (ULN), or at a dosage sufficient to maintain the subject's fasting blood ammonia level within a specified target range of 1 to 1.5, between 1 and 1.5, <1.5, or ≤1.5 times the ULN as described in PCT Application No. PCT/US13/71333 filed November 21, 2013 and entitled "Methods of Administering and Evaluating Nitrogen Scavenging Drugs for the Treatment of Hepatic Encephalopathy," which is hereby incorporated by reference in its entirety as if fully set forth herein. In certain embodiments, the upper limit of normal for blood ammonia is 35 µmol/L. In certain embodiments, the therapeutic intervention may be a nitrogen scavenging drug. In certain embodiments, the nitrogen scavenging drug may be selected from the group consisting of a PAA prodrug and sodium benzoate. In certain embodiments, the PAA prodrug may be selected from the group consisting of glyceryl tri-[4-phenylbutyrate] (HPN-100), phenylbutyric acid (PBA), sodium PBA (NaPBA), and a combination of two or more of HPN-100, PBA, and NaPBA.

The systems and methods provided herein for applying the disclosed HE episode grading system include written instruments and computer programs for use in applying the grading system. In certain embodiments, these systems and methods may comprise instructions to assist medical professionals in applying the grading system.

Screening Tools For Monitoring, Documenting, and Treating HE and Related Methods Thereof. Also provided herein in certain embodiments are screening tools for monitoring, documenting, and/or treating HE symptoms in a subject in need thereof and related methods thereof. In certain embodiments, the screening tools and related methods thereof include one or more set of steps for a user to perform. Since many HE episodes occur when a subject is at home or in some setting other than a health care provider's office or clinic, it is important that the caregivers of subjects with HE be familiar with the manifestations of HE episodes so that they can report them to a physician in an accurate and systematic manner to ensure that the subject receives the appropriate treatment. Thus, in certain embodiments, the user of the screening tools and related methods thereof may be a caregiver of a "subject in need thereof' as described herein. In certain embodiments, the caregiver may be a spouse, relative, friend, or some other non-medical person that has a relationship with the subject. As described in Example 2, the screening tools and methods thereof provided herein were developed based on concept elicitation interviews conducted with caregivers of patients with HE and were refined through cognitive interviews with caregivers of subjects with HE. As such, the screening tools and methods thereof incorporate concepts and language familiar to caregivers, which provides a user-friendly format for a caregiver that is uncomplicated and easy to understand. The screening tools provided herein, however, are intended only for use as screening tools and not as diagnostic tools, and are not considered substitutes for the caregiver's judgment as to when to seek medical care for the subject in need thereof.

As used herein, the terms "screening tool," "tool for screening," or "tool" may be used interchangeably.

As used herein, a "user" is a caregiver or a non-healthcare provider. In certain embodiments, the user may be instructed to perform or may perform the steps of the screening tools and related methods provided herein.

The screening tools and methods thereof as provided herein were developed to assist a caregiver with monitoring, documenting, and/or treating HE symptoms in a subject in need thereof. In certain embodiments, the screening tools and methods thereof were developed to provide a caregiver a system of determining whether a physician should be contacted and/or whether a subject should be administered a therapeutic intervention depending on certain criteria regarding the subject's mental and/or physical state. As provided in more detail below, the screening tools and methods thereof as provided herein may be used on a daily basis for monitoring the subject in need thereof. For example, in certain embodiments, the screening tools and methods thereof may be used as a daily diary for routinely monitoring the subject in need thereof. In other embodiments, the screening tools and methods thereof may be used because the subject is acting unusual. For example, in certain embodiments, the screening tools and methods thereof may be used because the subject is exhibiting unusual mental or physical behavior, or a combination thereof.

In certain embodiments, the tools for screening a subject in need thereof for HE symptoms and related methods thereof may comprise one or more of a set of steps. In certain embodiments, the one or more of a set of steps may comprise a first set of steps, a second set of steps, and/or a third set of steps for a user to perform. In certain embodiments, the sets of steps may be performed in an order as indicated. In certain embodiments, the sets of steps may be performed in any order. In certain embodiments, one or more of a set of steps may be skipped.

In certain embodiments, the first, second, and/or third set of steps of the screening tools and methods thereof each comprise one or more steps. Each of the steps within the first, second, and/or third set of steps are different. In certain embodiments, the steps may be performed in an order as indicated. In certain embodiments, the steps may be performed in any order. In certain embodiments, one or more steps may be skipped.

In certain embodiments, the one or more steps of the screening tools and methods thereof may include requiring that the user make one or more determinations about the subject. In certain embodiments, without limitation, one or more steps may require that the user make a determination about the subject's mental or physical state. For example, in certain embodiments, one step may require that the user make a determination about whether the subject has difficulty speaking. In another example, one step may require that the user make a determination about whether the subject is exhibiting unusual behavior.

In certain preferred embodiments, the steps of the screening tools and methods thereof may be presented in a questionnaire format. In certain embodiments, the steps may comprise one or more questions for the user to answer about the subject. Examples of questions for each of the steps are provided herein and in Tables 1 and 2. The questions should not be limited to the exact language provided in the examples; however, they should be structured such that they are intended to elicit the same type of information from the user.

In certain embodiments, the user may be provided with a selection of answers to the questions. In certain embodiments, the answers to the questions may be provided in any type of format known to one of ordinary skill in the art. For example, the answers to the questions may be, without limitation, restricted to a yes or no choice, provided in a multiple choice format by providing two or more answers for the user to choose, provided in an open-ended format where the user provides the answer, or any combination thereof.

In certain preferred embodiments, the screening tools and related methods thereof may be provided electronically. In certain embodiments, a branching logic algorithm may be utilized with the screening tools and related methods thereof such that specific steps may be provided to the user based on the user's purpose for using the screening tool. For example, the user may be provided one or more steps to perform if the user chooses to use the screening tool as a daily diary. Alternatively, if the user chooses to use the screening tool because the subject is exhibiting unusual mental or physical behavior, or a combination thereof, the user may be provided one or more steps to perform for this particular purpose. In certain embodiments, a branching logic algorithm may provide the user with particular steps based on the user's responses to previous steps. Determination of various criteria for the steps of the screening tool may be carried out by direct observation of and interaction with the subject in need thereof by the user.

In certain embodiments, the screening tools and related methods thereof may be provided to users on web-enabled devices. For example, a web-enabled device may be, without limitation, a computer, tablet, or smart phone. In certain embodiments, the screening tools and related methods thereof may be provided to users on web-enabled devices using a "bring your own device" (BYOD) approach. In other embodiments, the screening tools and related methods thereof may be provided in a paper format.

In certain embodiments, the screening tools and related methods thereof were developed to provide a user with a system of determining whether a physician should be contacted and/or whether a subject should be administered a therapeutic intervention (e.g., change in diet, alteration in dose of a prescribed medicine such as lactulose based on prior physician guidance, etc.) depending on whether certain criteria are met regarding the subject's mental and/or physical state. In certain embodiments, if one or more criteria are met as specified below, the user may contact or be instructed to contact a physician. In certain embodiments, if one or more criteria are met as specified below, the user may administer a therapeutic intervention to the subject. In certain embodiments, if one or more criteria are met as specified below, a party other than the user, such as a physician, may administer a therapeutic intervention to the subject. In certain embodiments, if one or more criteria are met as specified below and the subject was previously administered a dosage of a therapeutic intervention, the dosage of the therapeutic intervention may be modified and a modified dosage of the therapeutic intervention may be administered to the subject. In certain embodiments, the modified dosage may be a dosage that is greater than the dosage of therapeutic intervention that was previously administered to the subject. In certain embodiments, the therapeutic intervention may be lactulose. For example, if one or more criteria are met as specified below and the subject was previously administered a dosage of lactulose, the dosage of lactulose may be increased and the increased dosage of lactulose may be administered to the subject. In certain embodiments, the therapeutic intervention may be administered to the subject under the care of a physician. In certain embodiments, it is intended that the user administer and/or modify the dosage of the therapeutic intervention according to the recommendations of a physician. In certain embodiments, the therapeutic intervention may be modification of the subject's diet. For example, if one or more criteria are met as specified below and the subject was previously advised regarding diet, including the amount of protein to be regularly consumed by the subject, the amount of dietary protein consumed by the subject may be temporarily altered or lowered. In certain embodiments, the amount of dietary protein may be temporarily altered or lowered according to the recommendations of a physician.

In certain embodiments, if one or more criteria are met as specified below, a therapeutic intervention may be administered at a dosage sufficient to maintain a subject's fasting blood ammonia level at or below a specified threshold level of 1.5 times the upper limit of normal (ULN), or at a dosage sufficient to maintain the subject's fasting blood ammonia level within a specified target range of 1 to 1.5, between 1 and 1.5, <1.5, or ≤1.5 times the ULN as described in PCT Application No. PCT/US13/71333 filed November 21, 2013 and entitled "Methods of Administering and Evaluating Nitrogen Scavenging Drugs for the Treatment of Hepatic Encephalopathy." In certain embodiments, the upper limit of normal for blood ammonia is 35 µmol/L.

In certain embodiments, the term "therapeutic intervention" as used herein may be a nitrogen scavenging or ammonia binding agent such as a phenylacetate (PAA) prodrug (e.g., PBA, sodium PBA (Buphenyl®), glycerol phenylbutyrate (glyceryl tri-[4-phenylbutyrate], HPN-100, Ravicti®)) or sodium benzoate, L-omithine, L-aspartate (LOLA), a minimally absorbable or non-absorbable antibiotic (e.g., Rifaximin (Xifaxan®) or a polymorph thereof, neomycin), dietary protein and/or vitamin supplementation, a minimally absorbable or non-absorbable disaccharide (e.g., lactulose, lactitol), probiotics, flumazenil acarbose, an antimicrobial agent, hypertonic saline, mannitol, dexamethasone, thiopentone, indomethacin, an antiepileptic drug, N-acetylcysteine (NAC), a change in diet, or any combination thereof. In other embodiments, the therapeutic intervention may be a non-drug intervention such as intubation and mechanical ventilation, sedation, enema, liver transplant, or a liver assist device.

*Screening tools for monitoring, documenting, and*/*or treating HE symptoms on a daily basis and related methods thereof.* In certain embodiments, when the screening tools and related methods thereof are used on a daily basis for monitoring, documenting, and/or treating the HE symptoms of the subject in need thereof, a first, second, and/or third set of steps may be performed by a user. In certain embodiments, the user may be a caregiver of the subject. In certain embodiments, if the user performs the first set of steps, and none of the criteria are met from the first set of steps, then the user may skip performing the second set of steps and may perform the steps of the third set of steps.

In certain embodiments, the first set of steps may comprise one or more or steps selected from (i) determining whether the subject has unusual difficulty speaking, (ii) determining whether the subject is exhibiting unusual behavior, and (iii) determining whether the subject is more forgetful or confused than usual. In certain embodiments, the one or more steps in the first set of steps may be performed in any order. In certain embodiments, one or more steps in the first set of steps may be skipped.

In certain embodiments, step (i) comprises determining whether the subject in need thereof has unusual difficulty speaking. In certain embodiments, the user may be asked to determine whether the subject has unusual difficulty speaking. In certain embodiments, if the user determines that the subject has unusual difficulty speaking, then the criterion for step (i) has been met. In certain embodiments, the user may be asked a question regarding whether the subject has unusual difficulty speaking. For example, in certain embodiments, the user may be asked the question: Does the subject respond slowly to the user, repeat words, or slur words? In certain embodiments, if the answer to this question is yes, then the criterion for step (i) has been met. In certain embodiments, if the criterion for step (i) has been met, the user may perform one or more other steps in the first set of steps (i.e., steps (ii) or (iii) if they have not already been performed). For example, in certain preferred embodiments, if the criterion for step (i) has been met, the user may perform step (ii). In certain embodiments, if the criterion for step (i) has been met, the user may perform one or more steps in the second set of steps. In certain embodiments, if the criterion for step (i) has not been met (i.e., the subject does not have unusual difficulty speaking), the user may perform one or more other steps in the first set of steps, for example, the user may perform step (ii).

In certain embodiments, step (ii) comprises determining whether the subject is exhibiting unusual behavior. In certain embodiments, the user may be asked to determine whether the subject is exhibiting unusual behavior. In certain embodiments, if the user determines that the subject is exhibiting unusual behavior, then the criterion for step (ii) has been met. In certain embodiments, the user may be asked a question regarding whether the subject is exhibiting unusual behavior. For example, in certain embodiments, the user may be asked one or more of the questions: Is the subject moving more slowly; is the patient more angry or argumentative; or is the patient unable to perform daily tasks such as bathing, dressing, eating, or using the toilet? In certain embodiments, if the answer to any of these questions are yes, then the criterion for step (ii) has been met. In certain embodiments, if the criterion for step (ii) has been met, the user may perform one or more other steps in the first set of steps (i.e., steps (i) or (iii) if they have not already been performed). For example, in certain preferred embodiments, if the criterion for step (ii) has been met, the user may perform step (iii). In certain embodiments, if the criterion for step (ii) has been met, the user may perform one or more steps in the second set of steps. In certain embodiments, if the criterion for step (ii) has not been met (i.e., the subject is not exhibiting unusual behavior), the user may perform one or more other steps in the first set of steps, for example, the user may perform step (iii).

In certain embodiments, step (iii) comprises determining whether the patient is acting more forgetful or confused than usual. In certain embodiments, the user may be asked to determine whether the subject is acting more forgetful or confused than usual. In certain embodiments, if the user determines that the subject is acting more forgetful or confused than usual, then the criterion for step (iii) has been met. In certain embodiments, the user may be asked a question regarding whether the subject is acting more forgetful or confused than usual. For example, in certain embodiments, the user may be asked the question: Is the subject acting more forgetful or confused than usual? In certain embodiments, if the answer to this question is yes, then the criterion for step (iii) has been met. In certain embodiments, if the criterion for step (iii) has been met, the user may perform one or more other steps in the first set of steps (i.e., steps (i) or (ii) if they have not already been performed). In certain embodiments, if the criterion for step (iii) has been met, the user may perform one or more steps in the second set of steps. For example, in certain preferred embodiments, the user may perform step (iv).

In certain embodiments, if the criteria for steps (i), (ii), and (iii) have not been met, the user may perform one or more steps in the third set of steps. For example, the user may perform one or more of the steps (viii), (ix), and (x) as described herein.

In certain embodiments, a tool for screening a subject in need thereof for HE symptoms and related methods thereof may comprise a second set of steps. In certain embodiments, the second set of steps may be performed only if one or more criteria from the first set of steps are met. In certain embodiments, the second set of steps may comprise one or more or steps including (iv) determining whether the subject can stay awake when being spoken to, (v) determining whether the subject is disoriented as to person, (vi) determining whether the subject is disoriented as to place, and (vii) determining whether the subject is disoriented as to time. In certain embodiments, the one or more steps in the second set of steps may be performed in any order. In certain embodiments, one or more steps of the second set of steps may be skipped. In certain embodiments, if one or more criteria from the second set of steps are met, the user may contact or be instructed to contact a physician. In certain embodiments, if one or more criteria from the second set of steps are met, the user may administer a therapeutic intervention to the subject as described previously. In certain embodiments, if one or more criteria from the second set of steps are met, a party other than the user, such as a physician, may administer a therapeutic intervention to the subject. In certain embodiments, if one or more criteria from the second set of steps are met, and the subject was previously administered a dosage of a therapeutic intervention, the dosage of the therapeutic intervention may be modified and a modified dosage of the therapeutic intervention may be administered to the subject. In certain embodiments, the modified dosage may be a dosage that is greater than the dosage of therapeutic intervention that was previously administered to the subject. In certain embodiments, the therapeutic intervention may be lactulose. For example, if one or more criteria from the second set of steps are met, and the subject was previously administered a dosage of lactulose, the dosage of lactulose may be increased and the increased dosage of lactulose may be administered to the subject. In certain embodiments, the therapeutic intervention may be administered to the subject under the care of a physician. In certain embodiments, the therapeutic intervention may be administered at a dosage sufficient to maintain the subject's fasting blood ammonia level at or below a specified threshold of 1.5 times the upper limit of normal. In certain embodiments, the therapeutic intervention may be a nitrogen scavenging drug. In certain embodiments, the nitrogen scavenging drug may be selected from the group consisting of a PAA prodrug and sodium benzoate. In certain embodiments, the PAA prodrug may be selected from the group consisting of glyceryl tri-[4-phenylbutyrate] (HPN-100), phenylbutyric acid (PBA), sodium PBA (NaPBA), and a combination of two or more of HPN-100, PBA, and NaPBA.

In certain embodiments, step (iv) comprises determining whether the subject can stay awake when being spoken to. In certain embodiments, the user may be asked to determine whether the subject can stay awake when being spoken to. In certain embodiments, if the user determines that the subject cannot stay awake when being spoken to, then the criterion for step (iv) has been met. In certain embodiments, the user may be asked a question regarding whether the subject can stay awake when being spoken to. In certain embodiments, the user may be asked the question: Can the subject stay awake when you are talking to him/her? In certain embodiments, if the answer to this question is no, then the criterion for step (iv) has been met. In certain embodiments, if the criterion for step (iv) is met, the user may contact or be instructed to contact a physician. In certain embodiments, if the criterion for step (iv) is met, the user may be instructed to administer a therapeutic intervention to the subject. In certain embodiments, if the criterion for step (iv) is met, the subject may be administered a therapeutic intervention by the user or by some other party such as a physician as described previously. In certain embodiments, if the criterion for step (iv) has been met, the user may perform one or more other steps in the second set of steps (i.e., steps (v), (vi), or (vii) if they have not already been performed). For example, in certain preferred embodiments, the user may perform step (v). In certain embodiments, if the criterion for step (iv) has been met, the user may perform one or more steps in the third set of steps. In certain embodiments, if the criterion for step (iv) has not been met, the user may perform one or more other steps in the second set of steps (i.e., steps (v), (vi), or (vii) if they have not already been performed).

In certain embodiments, step (v) comprises determining whether the subject is disoriented as to person. In certain embodiments, the user may be asked to determine whether the subject is disoriented as to person. In certain embodiments, if the user determines that the subject is disoriented as to person, then the criterion for step (v) has been met. In certain embodiments, the user may be asked a question regarding whether the subject is disoriented as to person. For example, in certain embodiments, the user may be asked the question: Does the subject know who you are? In certain embodiments, the user the user may be asked the question: Does the subject know their own name and can the subject state their own name and/or can they recognize and name a present family member or user? In certain embodiments, if the answer to this question is no, then the criterion for step (v) has been met. In certain embodiments, if the criterion for step (v) has been met, the user may contact or be instructed to contact a physician. In certain embodiments, if the criterion for step (v) is met, the user may be instructed to administer a therapeutic intervention to the subject. In certain embodiments, if the criterion for step (v) has been met, the subject may be administered a therapeutic intervention as described previously. In certain embodiments, if the criterion for step (v) has been met, the user may perform one or more other steps in the second set of steps (i.e., steps (iv), (vi), or (vii) if they have not already been performed). For example, in certain preferred embodiments, the user may perform step (vi). In certain embodiments, if the criterion for step (v) has been met, the user may perform one or more steps in the third set of steps. In certain embodiments, if the criterion for step (v) has not been met, the user may perform one or more other steps in the second set of steps (i.e., steps (iv), (vi), or (vii) if they have not already been performed).

In certain embodiments, step (vi) comprises determining whether the subject is disoriented as to place. In certain embodiments, the user may be asked to determine whether the subject is disoriented as to place. In certain embodiments, if the user determines that the subject is disoriented as to place, then the criterion for step (vi) has been met. In certain embodiments, the user may be asked a question regarding whether the subject is disoriented as to place. For example, in certain embodiments, the user may be asked the question: Does the subject know what country or city or room or house he/she is in? In certain embodiments, the user may be asked the question: Does the subject know their present country, province/state, city/town, and/or type of place (e.g., hospital, house)? In certain embodiments, if the answer to this question is no, then the criterion for step (vi) has been met. In certain embodiments, if the criterion for step (vi) has been met, the user may contact or be instructed to contact a physician. In certain embodiments, if the criterion for step (vi) is met, the user may be instructed to administer a therapeutic intervention to the subject. In certain embodiments, if the criterion for step (vi) has been met, the subject may be administered a therapeutic intervention as described previously. In certain embodiments, if the criterion for step (vi) has been met, the user may perform one or more other steps in the second set of steps (i.e., steps (iv), (v), or (vii) if they have not already been performed). For example, in certain preferred embodiments, the user may perform step (vii). In certain embodiments, if the criterion for step (vi) has been met, the user may perform one or more steps in the third set of steps. In certain embodiments, if the criterion for step (vi) has not been met, the user may perform one or more other steps in the second set of steps (i.e., steps (iv), (v), or (vii) if they have not already been performed).

In certain embodiments, step (vii) comprises determining whether the subject is disoriented as to time. In certain embodiments, the user may be asked to determine whether the subject is disoriented as to time. In certain embodiments, if the user determines that the subject is disoriented as to time, then the criterion for step (vii) has been met. In certain embodiments, the user may be asked a question regarding whether the subject is disoriented as to time. For example, in certain embodiments, the user may be asked the question: Does the subject know what year or month or day of the week it is? In certain embodiments, if the answer to this question is no, then the criterion for step (vii) has been met. In certain embodiments, if the criterion for step (vii) has been met, the user may contact or may be instructed to contact a physician. In certain embodiments, if the criterion for step (vii) is met, the user may be instructed to administer a therapeutic intervention to the subject. In certain embodiments, if the criterion for step (vii) has been met, the subject may be administered a therapeutic intervention as described previously. In certain embodiments, if the criterion for step (vii) has been met, the user may perform one or more other steps in the second set of steps (i.e., steps (iv), (v), or (vi) if they have not already been performed). In certain embodiments, if the criterion for step (vii) has been met, the user may perform one or more steps in the third set of steps. For example, in certain embodiments, the user may perform step (viii). In certain embodiments, if the criterion for step (vii) has not been met, the user may perform one or more other steps in the second set of steps (i.e., steps (iv), (v), or (vi) if they have not already been performed) or one or more steps in the third set of steps (i.e., (viii), (ix), or (x)).

In certain embodiments, a tool for screening a subject in need thereof for HE symptoms and related methods thereof may comprise a third set of steps. In certain embodiments, the third set of steps may comprise one or more or steps including (viii) determining whether the subject took more lactulose in the day than his/her usual dose, (ix) determining whether the subject was taken to a physician, hospital, or whether emergency services were contacted, and (x) determining how many hours the user spent with the subject during that day. In certain embodiments, the one or more steps in the third set of steps may be performed in any order. In certain embodiments, one or more steps of the third set of steps may be skipped.

In certain embodiments, step (viii) comprises determining whether the subject took more lactulose in the day than his/her usual dose. In certain embodiments, the user may be asked to determine whether the subject took more lactulose in the day than his/her usual dose. In certain embodiments, if the user determines that the subject took more lactulose in the day than his/her usual dose, then the criterion for step (viii) has been met. In certain embodiments, the user may be asked a question regarding whether the subject took more lactulose in the day than his/her usual dose. For example, in certain embodiments, the user may be asked the question: Did the subject take more lactulose in the day than his/her usual dose? In certain embodiments, if the answer to this question is yes, then the criterion for step (viii) has been met. In certain embodiments, if the criterion for step (viii) has been met, the user may perform step (ix).

In certain embodiments, step (ix) comprises determining whether the subject was taken to a physician, hospital, or whether emergency services were contacted. In certain embodiments, the user may be asked to determine whether the subject was taken to a physician, hospital, or whether emergency services were contacted. In certain embodiments, the user may be asked a question regarding whether the subject was taken to a physician, hospital, or whether emergency services were contacted. For example, in certain embodiments, the user may be asked the question: Was the subject taken to a physician, hospital, or were emergency services contacted? In certain embodiments, the user may perform one or more steps in the third set of steps.

In certain embodiments, step (x) comprises determining how many hours the user spent with the subject during that day. In certain embodiments, the user may be asked to determine how many hours the user spent with the subject during that day. In certain embodiments, the user may be asked a question regarding how many hours the user spent with the subject during that day. For example, in certain embodiments, the user may be asked the question: How many hours did you spend with the patient today?

According to certain embodiments, a tool for screening a subject in need thereof for HE symptoms may comprise (a) a first set of steps comprising one or more steps selected from (i) determining whether the subject has unusual difficulty speaking, (ii) determining whether the subject is exhibiting unusual behavior, and (iii) determining whether the subject is more forgetful or confused than usual; and (b) a second set of steps comprising one or more steps selected from (iv) determining whether the subject can stay awake when being spoken to, (v) determining whether the subject is disoriented as to person, (vi) determining whether the subject is disoriented as to place, and (vii) determining whether the subject is disoriented as to time. In certain embodiments, the second set of steps may be performed only if one or more criteria from the first set of steps are met. In certain embodiments, a user may be instructed to contact a physician if one or more criteria from the second set of steps is met. In certain embodiments, a user may be the caregiver of the subject in need thereof. In certain embodiments, the steps may be provided in a questionnaire format. In certain embodiments, the tool may be provided in an electronic format with a branching logic algorithm. In certain embodiments, the tool may be provided on a web-enabled device. In certain embodiments, the steps may be performed on a daily basis to monitor the subject. In certain embodiments, the tool may be used at the same time each day. In certain embodiments, reminders may be sent to the user at the same time each day to remind the user to use the tool. In certain embodiments, the reminders may be sent to the user electronically. In certain embodiments, if one or more of the criteria are met from the second set of steps, the subject may be administered a therapeutic intervention as previously described. In certain embodiments, if one or more of the criteria are met from the second set of steps, the user may be instructed to or may administer a therapeutic intervention to the subject as previously described. In certain embodiments, the tool further comprises a third set of steps comprising one or more or steps including (viii) determining whether the subject took more lactulose in the day than his/her usual dose, (ix) determining whether the subject was taken to a physician, hospital, or whether emergency services were contacted, and (x) determining how many hours the user spent with the subject during that day.

According to certain embodiments, methods of treating HE symptoms in a subject in need thereof may comprise (a) a first set of steps comprising one or more steps selected from (i) determining whether the subject has unusual difficulty speaking, (ii) determining whether the subject is exhibiting unusual behavior, and (iii) determining whether the subject is more forgetful or confused than usual; and (b) a second set of steps comprising one or more steps selected from (iv) determining whether the subject can stay awake when being spoken to, (v) determining whether the subject is disoriented as to person, (vi) determining whether the subject is disoriented as to place, and (vii) determining whether the subject is disoriented as to time. In certain embodiments, the second set of steps may be performed only if one or more criteria from the first set of steps are met. In certain embodiments, the steps are performed by a user. In certain embodiments, the user may be a caregiver of the subject in need thereof. In certain embodiments, a physician may be contacted if one or more criteria are met from the second set of steps. In certain embodiments, the steps may be performed on a daily basis to monitor the subject in need thereof. In certain embodiments, the steps may be provided in a questionnaire format. In certain embodiments, the steps may be provided in an electronic format with a branching logic algorithm. In certain embodiments, the steps may be provided on a web-enabled device. In certain embodiments, reminders may be sent to the user at the same time each day to remind the caregiver to perform the steps of the method. In certain embodiments, the reminders may be sent electronically. In certain embodiments, the related methods described herein may also include methods of documenting HE symptoms in a subject in need thereof or methods of monitoring HE symptoms of a subject in need thereof.

*Screening tools for monitoring, documenting, and*/*or treating HE symptoms when the subject is exhibiting unusual behavior and related methods thereof.* In certain embodiments, the screening tools and related methods thereof may be used because the subject is exhibiting unusual mental or physical behavior, or a combination thereof. In certain embodiments, when the screening tools and methods thereof are used because the subject is exhibiting unusual mental or physical behavior, or a combination thereof, a first and/or second set of steps may be performed by a user. In certain embodiments, the user may be a caregiver of the subject.

In certain embodiments, when the screening tools and related methods thereof are used because the subject is exhibiting unusual mental or physical behavior, or a combination thereof, a first set of steps may be performed comprising one or more steps selected from (i) determining whether the subject can stay awake when being spoken to, (ii) determining whether the subject is disoriented as to person, (iii) determining whether the subject is disoriented as to place, and (iv) determining whether the subject is disoriented as to time. In certain embodiments, the one or more steps (i)-(iv) may be performed in any order. In certain embodiments, the one or more steps may be performed as indicated. In certain embodiments, one or more of steps (i)-(iv) may be skipped. In certain embodiments, if one or more criteria from the first set of steps are met, the user may contact or be instructed to contact a physician. In certain embodiments, if one or more criteria from the first set of steps are met, the user may administer a therapeutic intervention to the subject as described previously. In certain embodiments, if one or more criteria from the first set of steps are met, a party other than the user, such as a physician, may administer a therapeutic intervention to the subject. In certain embodiments, if one or more criteria from the first set of steps are met, and the subject was previously administered a dosage of a therapeutic intervention, the dosage of the therapeutic intervention may be modified and a modified dosage of the therapeutic intervention may be administered to the subject. In certain embodiments, the modified dosage may be a dosage that is greater than the dosage of therapeutic intervention that was previously administered to the subject. In certain embodiments, the therapeutic intervention may be lactulose. For example, if one or more criteria from the first set of steps are met, and the subject was previously administered a dosage of lactulose, the dosage of lactulose may be increased and the increased dosage of lactulose may be administered to the subject. In certain embodiments, the therapeutic intervention may be administered to the subject under the care of a physician. In certain embodiments, the therapeutic intervention may be administered at a dosage sufficient to maintain the subject's fasting blood ammonia level at or below a specified threshold of 1.5 times the upper limit of normal. In certain embodiments, the therapeutic intervention may be a nitrogen scavenging drug. In certain embodiments, the nitrogen scavenging drug may be selected from the group consisting of a PAA prodrug and sodium benzoate. In certain embodiments, the PAA prodrug may be selected from the group consisting of glyceryl tri-[4-phenylbutyrate] (HPN-100), phenylbutyric acid (PBA), sodium PBA (NaPBA), and a combination of two or more of HPN-100, PBA, and NaPBA.

In certain embodiments, step (i) comprises determining whether the subject can stay awake when being spoken to. In certain embodiments, the user may be asked to determine whether the subject can stay awake when being spoken to. In certain embodiments, if the user determines that the subject cannot stay awake when being spoken to, then the criterion for step (i) has been met. In certain embodiments, the user may be asked a question regarding whether the subject can stay awake when being spoken to. In certain embodiments, the user may be asked the question: Can the subject stay awake when you are talking to him/her? In certain embodiments, if the answer to this question is no, then the criterion for step (i) has been met. In certain embodiments, if the criterion for step (i) is met, the user may contact or be instructed to contact a physician. In certain embodiments, if the criterion for step (i) is met, the user may be instructed to administer a therapeutic intervention to the subject. In certain embodiments, if the criterion for step (i) is met, the subject may be administered a therapeutic intervention as previously described. In certain embodiments, if the criterion for step (i) has been met, the user may perform one or more other steps in the first set of steps (i.e., steps (ii), (iii), or (iv) if they have not already been performed). For example, in certain preferred embodiments, the user may perform step (ii). In certain embodiments, if the criterion for step (i) has not been met, the user may perform one or more other steps in the first set of steps (i.e., steps (ii), (iii), or (iv) if they have not already been performed).

In certain embodiments, step (ii) comprises determining whether the subject is disoriented as to person. In certain embodiments, the user may be asked to determine whether the subject is disoriented as to person. In certain embodiments, if the user determines that the subject is disoriented as to person, then the criterion for step (ii) has been met. In certain embodiments, the user may be asked a question regarding whether the subject is disoriented as to person. For example, in certain embodiments, the user may be asked the question: Does the subject know who you are? In certain embodiments, the user the user may be asked the question: Does the subject know their own name and can the subject state their own name and/or can they recognize and name a present family member or user? In certain embodiments, if the answer to this question is no, then the criterion for step (ii) has been met. In certain embodiments, if the criterion for step (ii) has been met, the user may contact or be instructed to contact a physician. In certain embodiments, if the criterion for step (ii) is met, the user may be instructed to administer a therapeutic intervention to the subject. In certain embodiments, if the criterion for step (ii) has been met, the subject may be administered a therapeutic intervention as previously described. In certain embodiments, if the criterion for step (ii) has been met, the user may perform one or more other steps in the first set of steps (i.e., steps (i), (iii), or (iv) if they have not already been performed). For example, in certain preferred embodiments, the user may perform step (iii). In certain embodiments, if the criterion for step (ii) has not been met, the user may perform one or more other steps in the first set of steps (i.e., steps (i), (iii), or (iv) if they have not already been performed).

In certain embodiments, step (iii) comprises determining whether the subject is disoriented as to place. In certain embodiments, the user may be asked to determine whether the subject is disoriented as to place. In certain embodiments, if the user determines that the subject is disoriented as to place, then the criterion for step (iii) has been met. In certain embodiments, the user may be asked a question regarding whether the subject is disoriented as to place. For example, in certain embodiments, the user may be asked the question: Does the subject know what country or city or room or house he/she is in? In certain embodiments, the user may be asked the question: Does the subject know their present country, province/state, city/town, and/or type of place (e.g., hospital, house)? In certain embodiments, if the answer to this question is no, then the criterion for step (iii) has been met. In certain embodiments, if the criterion for step (iii) has been met, the user may contact or be instructed to contact a physician. In certain embodiments, if the criterion for step (iii) is met, the user may be instructed to administer a therapeutic intervention to the subject. In certain embodiments, if the criterion for step (iii) has been met, the subject may be administered a therapeutic intervention as previously described. In certain embodiments, if the criterion for step (iii) has been met, the user may perform one or more other steps in the first set of steps (i.e., steps (i), (ii), or (iv) if they have not already been performed). For example, in certain preferred embodiments, the user may perform step (iv). In certain embodiments, if the criterion for step (iii) has not been met, the user may perform one or more other steps in the first set of steps (i.e., steps (i), (ii), or (iv) if they have not already been performed).

In certain embodiments, step (iv) comprises determining whether the subject is disoriented as to time. In certain embodiments, the user may be asked to determine whether the subject is disoriented as to time. In certain embodiments, if the user determines that the subject is disoriented as to time, then the criterion for step (iv) has been met. In certain embodiments, the user may be asked a question regarding whether the subject is disoriented as to time. For example, in certain embodiments, the user may be asked the question: Does the subject know what year or month or day of the week it is? In certain embodiments, if the answer to this question is no, then the criterion for step (iv) has been met. In certain embodiments, if the criterion for step (iv) has been met, the user may contact or may be instructed to contact a physician. In certain embodiments, if the criterion for step (iv) is met, the user may be instructed to administer a therapeutic intervention to the subject. In certain embodiments, if the criterion for step (iv) has been met, the subject may be administered a therapeutic intervention as previously described. In certain embodiments, if the criterion for step (iv) has been met, the user may perform one or more other steps in the first set of steps (i.e., steps (i), (ii), or (iii) if they have not already been performed), or the user may perform one or more steps in the second set of steps. In certain embodiments, if the criterion for step (iv) has not been met, the user may perform one or more other steps in the first set of steps (i.e., steps (i), (ii), or (iii) if they have not already been performed). In certain embodiments, if the criterion for step (iv) has not been met, the user may perform one or more steps in the second set of steps.

In certain embodiments, a tool for screening a subject in need thereof for HE symptoms and related methods thereof may comprise a second set of steps. In certain embodiments, the second set of steps may comprise one or more or steps including (v) determining whether the subject took more lactulose in the day than his/her usual dose, (vi) determining whether the subject was taken to a physician, hospital, or whether emergency services were contacted, and (vii) determining how many hours the user spent with the subject during that day. In certain embodiments, the one or more steps in the second set of steps may be performed in any order. In certain embodiments, one or more steps of the second set of steps may be skipped.

In certain embodiments, step (v) comprises determining whether the subject took more lactulose in the day than his/her usual dose. In certain embodiments, the user may be asked to determine whether the subject took more lactulose in the day than his/her usual dose. In certain embodiments, if the user determines that the subject took more lactulose in the day than his/her usual dose, then the criterion for step (v) has been met. In certain embodiments, the user may be asked a question regarding whether the subject took more lactulose in the day than his/her usual dose. For example, in certain embodiments, the user may be asked the question: Did the subject take more lactulose in the day than his/her usual dose? In certain embodiments, if the answer to this question is yes, then the criterion for step (v) has been met. In certain embodiments, if the criterion for step (v) has been met, the user may perform step (vi).

In certain embodiments, step (vi) comprises determining whether the subject was taken to a physician, hospital, or whether emergency services were contacted. In certain embodiments, the user may be asked to determine whether the subject was taken to a physician, hospital, or whether emergency services were contacted. In certain embodiments, the user may be asked a question regarding whether the subject was taken to a physician, hospital, or whether emergency services were contacted. For example, in certain embodiments, the user may be asked the question: Was the subject taken to a physician, hospital, or were emergency services contacted? In certain embodiments, if the criterion for step (vi) has been met, the user may perform step (vii).

In certain embodiments, step (vii) comprises determining how many hours the user spent with the subject during that day. In certain embodiments, the user may be asked to determine how many hours the user spent with the subject during that day. In certain embodiments, the user may be asked a question regarding how many hours the user spent with the subject during that day. For example, in certain embodiments, the user may be asked the question: How many hours did you spend with the patient today?

According to certain embodiments, a tool for screening a subject in need thereof for HE symptoms may comprise one or more of the following steps: (i) determining whether the subject can stay awake when being spoken to, (ii) determining whether the subject is disoriented as to person, (iii) determining whether the subject is disoriented as to place, and (iv) determining whether the subject is disoriented as to time. In certain embodiments, a user may be instructed to contact a physician if one or more criteria in steps (i)-(iv) are met. In certain embodiments, the user may be a caregiver of the subject in need thereof. In certain embodiments, steps (i)-(iv) are performed because the subject is exhibiting unusual mental or physical behavior, or a combination thereof. In certain embodiments, the steps of the tool may be provided in a questionnaire format. In certain embodiments, the tool may be provided in an electronic format with a branching logic algorithm. In certain embodiments, the tool may be provided on a web-enabled device. In certain embodiments, the user may be instructed to administer a therapeutic intervention to the subject in need thereof if one or more criteria in steps (i)-(iv) are met. In certain embodiments, the subject in need thereof may be administered a therapeutic intervention as provided herein if one or more criteria in steps (i)-(iv) are met. In certain embodiments, the tool may further comprise one or more of the following steps including (v) determining whether the subject took more lactulose in the day than his/her usual dose, (vi) determining whether the subject was taken to a physician, hospital, or whether emergency services were contacted, and (vii) determining how many hours the user spent with the subject during that day.

According to certain embodiments, a method of treating HE symptoms in a subject in need thereof may comprise one or more of the following steps: (i) determining whether the subject can stay awake when being spoken to, (ii) determining whether the subject is disoriented as to person, (iii) determining whether the subject is disoriented as to place, and (iv) determining whether the subject is disoriented as to time. In certain embodiments, a physician is contacted if one or more of the criteria in steps (i)-(iv) are met. In certain embodiments, a user of the subject in need thereof may perform one or more of steps (i)-(iv). In certain embodiments, the user may be a caregiver of the subject in need thereof. In certain embodiments, the one or more of steps (i)-(iv) are performed because the subject is exhibiting unusual mental or physical behavior, or a combination thereof. In certain embodiments, the steps may be provided in a questionnaire format. In certain embodiments, the steps may be provided in an electronic format with a branching logic algorithm. In certain embodiments, the steps may be provided on a web-enabled device. In certain embodiments, the method may further comprise administering a therapeutic intervention to the subject in need thereof if one or more criteria in steps (i)-(iv) are met. In certain embodiments, the method may further comprise one or more of the following steps including (v) determining whether the subject took more lactulose in the day than his/her usual dose, (vi) determining whether the subject was taken to a physician, hospital, or whether emergency services were contacted, and (vii) determining how many hours the user spent with the subject during that day. In certain embodiments, the related methods described herein may also include methods of documenting HE symptoms in a subject in need thereof or methods of monitoring HE symptoms of a subject in need thereof.

According to certain embodiments provided herein, kits are provided comprising the screening tools as described herein to assist a caregiver with monitoring, documenting, and/or treating HE symptoms in a subject in need thereof. In certain embodiments, methods are contemplated comprising the use of the kits described herein.

One of ordinary skill in the art will recognize that the various embodiments described herein can be combined.

The following examples are provided to better illustrate the claimed invention.

### Examples

### Example 1: Diagnosis and classification/grading of HE episodes:

A subject with a documented history of cirrhosis and HE is evaluated to determine whether they are experiencing an overt HE episode based on the following determinations. Each of the steps below may be carried out by a medical professional in a clinical or non-clinical setting. In certain situations, the steps may be carried out by reviewing the subject's medical records. Determination of the duration of various criteria is carried out by direct observation of the subject by medical personnel or by interview of the caregiver and/or persons accompanying the subject.

### Step 1: Disorientation as to time:

The subject is tested to determine whether they know the year, month, day of the week, and/ date of the month. Each question is asked only one time, and the subject is given sufficient time to answer. A single prompt is allowed for questions relating to whether the subject knows the year. If the subject either does not know the answer to any two or more of these questions or does not know the year, they are deemed disoriented as to time, and are classified as experiencing an overt HE episode. In certain embodiments, treatment is initiated or continued based on this classification.

### Step 2: Disorientation as to place:

The subject is tested to determine whether they know their present country, province/state, city/town, and/or type of place (e.g., hospital, house). Each question is asked only one time, and the subject is given sufficient time to answer. If the subject does not know the answer to any one or more of these items, they are deemed disoriented as to place, and are classified as experiencing an overt HE episode (grade 2). In certain embodiments, treatment is initiated or continued based on this classification.

### Step 3: Disorientation as to person:

The subject is tested to determine whether they know and can state their own name and/or whether they recognize and can name a present family member or caregiver. Each question is asked only one time, and the subject is given sufficient time to answer. If the subject does not know the answer to any one or more of these items, they are deemed disoriented as to person, and are classified as experiencing an overt HE episode (grade 2). In certain embodiments, treatment is initiated or continued based on this classification.

### Step 4: Asterixis and lethargy:

The subject is evaluated for asterixis by having them extend their arms with wrists flexed backwards and fingers open for 30 seconds or more. The subject is deemed to exhibit asterixis if they demonstrate at least three flaps in a 30 second period.

The subject is evaluated for lethargy by determining whether they inattentive and sleepy, but awaken when spoken to.

If the subject exhibits both asterixis and lethargy, they are classified as experiencing an overt HE episode (grade 2). In certain embodiments, treatment is initiated or continued based on this classification.

### Step 5: Combined disorientation:

If the subject is deemed disoriented as to time, place, and person in steps 1-3, they are classified as experiencing an overt HE episode (at least a grade 3). In certain embodiments, treatment is initiated or continued based on this classification.

### Step 6: Somnolence:

The subject is evaluated for somnolence by determining whether they are asleep but temporarily arousable in response to verbal or physical stimulation. If the subject exhibits somnolence, they are classified as experiencing an overt HE episode (at least a grade 3). In certain embodiments, treatment is initiated or continued based on this classification.

### Step 7: Comatose:

The subject is evaluated to determine whether they are comatose. If they are comatose, they are classified as experiencing an overt HE episode (grade 4). In certain embodiments, treatment is initiated or continued based on this classification.

In certain cases, one or more of the steps above may be skipped. For example, where the subject is comatose, they may be classified as experiencing an overt HE episode (at least a grade 4) without performing one or more of steps 1-6.

Subjects classified as experiencing a grade 2, 3, or 4 HE episode (overt HE) using the steps set forth above may be treated by administering one or more therapeutic interventions. The specific therapeutic intervention(s) may vary depending on the grade, as well as on other factors such as the subject's age or general health.

### Example 2: Caregiver's electronic screening tool for monitoring patients with liver cirrhosis complicated by overt HE:

A patient with HE may exhibit altered mental and/or physical signs and symptoms likely due to increased blood ammonia levels. Frequently, patients with HE may not even realize that they have HE because of their altered mental status. Many of a patient's HE episodes occur when the patient is at home or in some setting other than a clinical setting. Thus, it is often the patient's caregiver (i.e., spouse, relative, or friend) who first recognizes the patient's altered mental state or behavior. It is therefore important for the caregiver to be familiar with the signs and symptoms of HE so that they know when to alert a physician so that the patient can receive the appropriate treatment.

A screening tool was developed to assist a caregiver with monitoring a patient with liver disease, such as cirrhosis, and a documented history of HE, and to standardize the daily monitoring of the patient. The screening tool was developed based on concept elicitation interviews conducted with caregivers of patients with HE and was refined through cognitive interviews with caregivers of patients with HE. Therefore, the screening tool incorporated concepts and language familiar to caregivers. The screening tool captured the same concepts as presented in Example 1 regarding diagnosis and classification/grading of HE episodes, such as orientation and level of consciousness, but also included other concepts that are meaningful to the caregivers, e.g., changes in the activities of the patient's daily life. The usability of the screening tool was subsequently tested under "real world" conditions by caregivers. The screening tool was provided to caregivers on web-enabled devices using a "bring your own device" approach.

Each of the steps of the screening tool was carried out by a caregiver or some third party. The steps were provided as a questionnaire in an electronic format with questions for the caregiver to answer. The caregiver was first given the option of selecting whether he/she was using the screening tool for the purposes of completing the "daily diary" (i.e., routine completion of the daily diary about the same time each day based on observing the patient) or "because the patient is acting unusual." A branching logic algorithm was utilized such that different questions were displayed to the caregiver based on the caregiver's purpose for using the screening tool. For example, if the caregiver selected the "daily diary", the caregiver would be provided questions specific to the "daily diary." Additionally, the branching logic algorithm took the caregiver down a particular response path based on the caregiver's responses to previous questions. Determination of various criteria for the steps of the screening tool was carried out by direct observation of and interaction with the patient by the caregiver.

This screening tool was used as a "daily diary" for the caregiver to monitor the patient's activity and mental state on a daily basis. Additionally, since it is often the caregiver who alerts the patient's physician to changes in the patient's physical and mental state, this screening tool was also used in situations when the caregiver believed that "the patient was acting unusual" to help the caregiver determine whether the patient's symptoms were such that a physician should be contacted. The screening tool, however, was intended for use only as a screening tool and not as a diagnostic tool, and was not considered a substitute for the caregiver's judgment as to when to seek medical care for the patient with HE.

*Use of the screening tool as a "daily diary"*: Reminders were sent to the caregivers electronically at the same time on a daily basis to remind the caregiver to complete the "daily diary." The caregiver completed the "daily diary" at approximately the same time each day and answered the questions based on observations of the patient. Examples of the various steps that were presented to the caregiver when "daily diary" was selected are as follows:

### Step 1: Time spent with the patient:

The caregiver was asked to provide the amount of hours he/she has spent with the patient during that day.

### Step 2: Speech:

The caregiver was asked whether the patient has unusual difficulty speaking. For example, does the patient respond slowly to the caregiver, repeat words, or slur words?

### Step 3: Physical actions:

The caregiver was asked to determine whether the patient is acting in an unusual manner. For example, is the patient moving more slowly; is the patient more angry or argumentative; or is the patient unable to perform daily tasks such as bathing, dressing, eating, or using the toilet?

### Step 4: Memory:

The caregiver was asked whether the patient is acting more forgetful or confused than usual.

Based on the caregiver's answers in steps 2-4, if the answer to all of the questions was "no", then the daily diary skipped to step 9 of the questionnaire provided below. However, if the answer to any of the questions in steps 2-4 was "yes", the daily diary automatically continued with the steps in the questionnaire.

### Step 5: Lethargy:

The caregiver was asked whether the patient could stay awake when the caregiver is talking to him/her.

### Step 6: Disorientation as to person:

The caregiver was asked whether the patient knows and can state their own name and/or whether they recognize and can name a present family member or caregiver. If the patient does not know the answer to any one or more of these items, the patient was deemed disoriented as to person.

### Step 7: Disorientation as to place:

The caregiver was asked whether the patient knows their present country, province/state, city/town, and/or type of place (e.g., hospital, house). If the patient does not know the answer to any one or more of these items, the patient was deemed disoriented as to place.

### Step 8: Disorientation as to time:

The caregiver was asked whether the patient knows the year, month, day of the week, and/ date of the month. If the patient does not know the answer to any of these items, the patient was deemed disoriented as to time.

Based on the caregiver's answers in steps 5-8, if the answer to any of the questions in steps 5-8 was "no", the daily diary automatically continued to steps 9 and 10 of the questionnaire and the caregiver was instructed to contact a physician.

### Step 9: Dosing:

The caregiver was asked whether the patient has taken more lactulose than his/her usual dose on that particular day.

### Step 10: Physician contact:

The caregiver was asked whether he/she has initiated contact with a medical professional (i.e., whether the caregiver has contacted emergency services or has taken the patient to the doctor or hospital).

An example of the steps that were provided to caregivers for the "daily diary" screening tool is provided in Table 1. The question flow in Table 1 was provided when the caregiver selected that they were using the screening tool as party of their routine diary completion.

**Table 1: Example question flow for the caregiver's "daily diary":**

| **Question flow if "Daily diary completion" is selected** | | |
|---|---|---|
| 1. How many hours did you spend with the patient today? | _ **hours** | |
| 2. Does the patient have unusual difficulty speaking (for example, responds slowly, repeats words, or slurs words)? | **□ No** | **□ Yes** |
| 3. Is the patient acting unusual (for example, moves more slowly; is more angry or argumentative; is unable to perform daily tasks such as bathing, dressing, eating, or using the toilet)? | **□ No** | **□ Yes** |
| 4. Is the patient more forgetful or confused than usual? | **□ No** | **□ Yes** |
| | **If ALL above answers are No, skip to #9** | **If ANY above answers are Yes, continue** |
| 5. Can the patient stay awake when you are talking to him/her? | **□ Yes** | **□ No** |
| 6. Does the patient know who you are? | **□ Yes** | **□ No** |
| 7. Does the patient know what country or city or room in the house he/she is in? | **□ Yes** | **□ No** |
| 8. Does the patient know what year or month or day of the week it is? | **□ Yes** | **□ No** |
| | | **If ANY answers to questions 5-8 are No, complete the items below and call the study doctor** |
| 9. Did the patient take more lactulose today than his/her usual dose? | **□ No** | **□ Yes** |
| 10. Did you take the patient to the doctor or hospital or call emergency services? | **□ No** | **□ Yes** |

One or more of the steps listed in Table 1 can be skipped. For example, if the caregiver selected "no" to any of the steps 5-8, the caregiver could contact a physician immediately without performing any of the other remaining steps. A therapeutic intervention may be administered to patients if the caregiver selected "no" to any of the steps 5-8.

*Use of the screening tool "because the patient is acting unusual":* Additionally, the caregiver was given the option of using the screening tool "because the patient is acting unusual." The screening tool was used to assist the caregiver in evaluating the behavioral activities of a patient with a severe liver disease, such as cirrhosis, or a documented history of HE when "the patient is acting unusual." This was useful in determining whether the caregiver should contact a physician when the caregiver believes that the patient is exhibiting unusual behavioral activity. Examples of the various steps that were presented to the caregiver when "Because the patient is acting unusual" was selected are as follows:

### Step 1: Lethargy:

The caregiver was asked whether the patient is inattentive and sleepy, but can stay awake when the caregiver is talking to him/her.

### Step 2: Disorientation as to person:

The caregiver was asked whether the patient knows and can state their own name and/or whether they recognize and can name a present family member or caregiver. If the patient does not know the answer to any one or more of these items, the patient was deemed disoriented as to person.

### Step 3: Disorientation as to place:

The caregiver was asked whether the patient knows their present country, province/state, city/town, and/or type of place (e.g., hospital, house). If the patient does not know the answer to any one or more of these items, the patient was deemed disoriented as to place.

### Step 4: Disorientation as to time:

The caregiver was asked whether the patient knows the year, month, day of the week, and/ date of the month. If the patient does not know the answer to any of these items, the patient was deemed disoriented as to time.

### Step 5: Time spent with the patient:

The caregiver was asked to provide the amount of hours he/she has spent with the patient during that day.

### Step 6: Dosing:

The caregiver was asked whether the patient has taken more lactulose than his/her usual dose on that particular day.

### Step 7: Physician contact:

The caregiver was asked whether he/she has initiated contact with a medical professional (i.e., whether the caregiver has contacted emergency services or has taken the patient to the doctor or hospital).

Based on the caregiver's answers in steps 1-4, if the answer to any of the questions in steps 1-4 was "no", the caregiver was instructed to contact a physician.

An example of the steps that were provided to caregivers for the purpose of "because the patient is acting usual" is provided in Table 2. The question flow in Table 1 was provided when the caregiver selected that they were using the screening tool "because the patient is acting unusual."

**Table 2: Example question flow for "Because the patient is acting usual":**

| **Question flow if "Because the patient is acting unusual" is selected** | | |
|---|---|---|
| 1. Can the patient stay awake when you are talking to him/her? | **□ Yes** | **□ No** |
| 2. Does the patient know who you are? | **□ Yes** | **□ No** |
| 3. Does the patient know what country or city or room in the house he/she is in? | **□ Yes** | **□ No** |
| 4. Does the patient know what year or month or day of the week it is? | **□ Yes** | **□ No** |
| 5. How many hours did you spend with the patient today? | **__hours** | |
| 6. Did the patient take more lactulose today than his/her usual dose? | **□ No** | **□ Yes** |
| 7. Did you take him/her to the doctor or hospital or call emergency services? | **□ No** | **□ Yes** |
| **CALL THE STUDY DOCTOR IF YOU ANSWERED NO TO ANY OF THE QUESTIONS 1-4** | | |

One or more of the steps listed in Table 2 can be skipped. For example, if the caregiver selected "no" to any of the steps 1-4, the caregiver may contact a physician immediately without performing any of the other remaining steps. A therapeutic intervention may be administered to patients if the caregiver selected "no" to any of the steps 1-4.

As stated above, the foregoing is intended to illustrate various embodiments of the present invention.

### REFERENCES

1. Amodio J Hepatol 49:346 (2008)
2. Bajaj Aliment Pharmacol Ther 33:739 (2011)
3. Blei Am J Gastroenterol 96:1968 (2001)
4. Conn Gastroenterology 72(4 Pt 1):573 (1977)
5. Ferenci Hepatology 35:716 (2002)
6. Hassanein Hepatology 46:1853 (2007)
7. Hassanein Dig Dis Sci 53:529 (2008)
8. Hassanein Am J Gastroenterol 104:1392 (2009)
9. Ortiz Aliment Pharmacol Ther 26:859 (2007)
10. Parsons-Smith Lancet 273:867 (1957)
11. Salam Aliment Pharmacol Ther 35:913 (2012)
12. Vilstrup Hepatology 2:715 (2014)

## Claims

1. Glyceryl tri-[4-phenylbutyrate] (HPN-100) for use in a method of treating an overt hepatic encephalopathy (HE) episode in an adult or pediatric human subject suspected to be currently experiencing an HE episode, wherein the glyceryl tri-[4-phenylbutyrate] (HPN-100) is administered to the subject if the subject is classified as experiencing an overt HE episode;
wherein the subject is classified as experiencing an overt HE episode if the subject meets only one of the criteria set forth in (a), (b), or (c) :
(a) determining whether the subject has been disoriented as to time, place, or person for at least one hour;
(b) determining whether the subject has been lethargic for at least one hour and is exhibiting asterixis; or
(c) determining that the subject is incapable of being assessed due to disorientation as to time, place, and person, somnolence, or coma,
wherein the glyceryl tri-[4-phenylbutyrate] (HPN-100) is administered at a dosage sufficient to maintain the subject's fasting blood ammonia level at or below a specified threshold of 1.5 times the upper limit of normal.

2. Glyceryl tri-[4-phenylbutyrate] (HPN-100) for use as in claim 1, wherein the upper limit of normal for blood ammonia is 35 µmοl/L.

## Patentansprüche

1. Glyceryl-tri-[4-phenylbutyrat] (HPN-100) zur Verwendung bei einem Verfahren zur Behandlung einer Overt-HE(Hepatische Enzephalopathie)-Episode bei einem menschlichen Individuum im Erwachsenen- oder Kindesalter, bei dem vermutet wird, dass es gegenwärtig eine HE-Episode durchmacht, wobei das Glyceryl-tri-[4-phenylbutyrat] (HPN-100) dem Individuum verabreicht wird, falls das Individuum als eine Overt-HE-Episode durchmachend klassifiziert wird;
wobei das Individuum als eine Overt-HE-Episode durchmachend klassifiziert wird, falls das Individuum nur eines der Kriterien gemäß (a), (b) oder (c) erfüllt:
(a) Bestimmen, ob das Individuum wenigstens eine Stunde hinsichtlich Zeit, Ort oder Person verwirrt war;
(b) Bestimmen, ob das Individuum wenigstens eine Stunde lethargisch war und Asterixis zeigt; oder
(c) Bestimmen, dass das Individuum aufgrund von Verwirrung hinsichtlich Zeit, Ort und Person, Somnolenz oder Koma nicht beurteilt werden kann, wobei das Glyceryl-tri-[4-phenylbutyrat] (HPN-100) in einer Dosierung verabreicht wird, die hinreichend ist, um den Fasten-Blutammoniakspiegel des Individuums auf oder unter einem angegebenen Schwellenwert von 1,5 mal dem oberen Grenzwert des Normalbereichs zu halten.

2. Glyceryl-tri-[4-phenylbutyrat] (HPN-100) zur Verwendung nach Anspruch 1, wobei der obere Grenzwert des Normalbereichs für Blutammoniak bei 35 µmol/l liegt.

## Revendications

1. Tri-[4-phénylbutyrate] de glycéryle (HPN-100) pour utilisation dans un procédé de traitement d'un épisode manifeste d'encéphalopathie hépatique (EH) chez un sujet humain adulte ou pédiatrique suspecté de subir actuellement un épisode d'EH, le tri-[4-phénylbutyrate] de glycéryle (HPN-100) étant administré au sujet si le sujet est classé comme subissant un épisode d'EH manifeste ;
le sujet étant classé comme subissant un épisode d'EH manifeste si le sujet satisfait à un seul des critères décrits dans (a), (b), ou (c) :
(a) détermination que le sujet a été ou non désorienté en termes d'heure, de lieu ou de personne pendant au moins une heure ;
(b) détermination que le sujet a été ou non léthargique pendant au moins une heure et présente un astérixis ; ou
(c) détermination que le sujet ne peut pas être évalué en raison de la désorientation en termes d'heure, de lieu et de personne, d'une somnolence ou d'un coma,
le tri-[4-phénylbutyrate] de glycéryle (HPN-100) étant administré à une dose suffisante pour maintenir un taux sanguin d'ammoniac à jeun du sujet inférieur ou égal à un seuil spécifié de 1,5 fois la limite supérieure de la normale.

2. Tri-[4-phénylbutyrate] de glycéryle (HPN-100) pour utilisation selon la revendication 1, la limite supérieure de la normale pour l'ammoniac sanguin étant de 35 µmol/l.
